(19) Europäisches Patentamt
European Patent Office
Office européen des brevets

(11) EP 4 023 680 A1

(12) **EUROPEAN PATENT APPLICATION**
published in accordance with Art. 153(4) EPC

(43) Date of publication:
**06.07.2022 Bulletin 2022/27**

(21) Application number: **20870635.8**

(22) Date of filing: **02.10.2020**

(51) International Patent Classification (IPC):
***C08B 30/14** (2006.01)* ***A23L 29/212** (2016.01)*

(52) Cooperative Patent Classification (CPC):
**A23L 29/212; C08B 30/14**

(86) International application number:
**PCT/JP2020/037627**

(87) International publication number:
**WO 2021/066168 (08.04.2021 Gazette 2021/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.10.2019 JP 2019182147**

(71) Applicant: **San-Ei Gen F.F.I., INC.**
**Toyonaka-shi, Osaka 561-8588 (JP)**

(72) Inventors:
- **ISHIHARA,Sayaka**
  **Toyonaka-shi, Osaka 561-8588 (JP)**
- **NAKAUMA,Makoto**
  **Toyonaka-shi, Osaka 561-8588 (JP)**
- **OTA,Miki**
  **Toyonaka-shi, Osaka 561-8588 (JP)**

(74) Representative: **Cabinet Beau de Loménie**
**158, rue de l'Université**
**75340 Paris Cedex 07 (FR)**

# (54) METHOD FOR PRODUCING PHYSICALLY MODIFIED STARCH

(57) The present disclosure relates to providing a modified starch that exhibits favorable viscosity by heating and is less likely to decrease in viscosity even by high temperature heating, or has excellent viscosity stability after gelatinization. The modified starch is obtained by preparing a mixture for heat treatment containing starch and a plant-derived dietary fiber or a mushroom-derived dietary fiber and having a specific moisture content, and then dry heating the mixture, wherein the pH at 25°C when 5 g of the mixture for heat treatment is suspended in 95 ml of water is 5 to 12.

FIG. 2

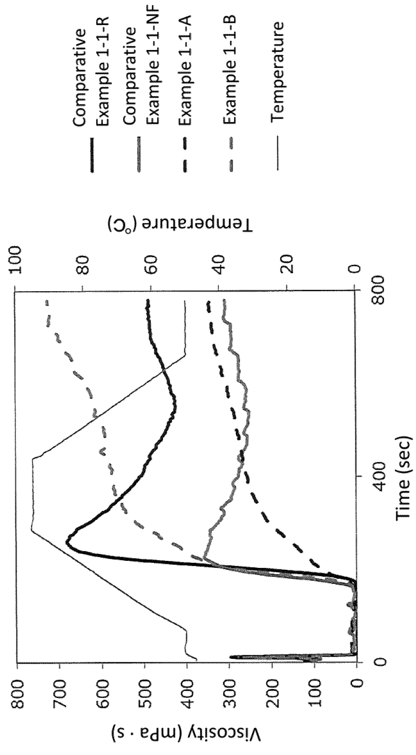


EP 4 023 680 A1

## Description

TECHNICAL FIELD

The present invention relates to a method for producing a physically modified starch, a method for suppressing viscosity decrease at the time of gelatinization of starch or by subsequent high temperature heating, or imparting viscosity stability after gelatinization, an agent for physically modifying starch, a physically modified starch, and a formulation and food or beverage product using the same.

BACKGROUND ART

When heated in the presence of a large amount of water, starch absorbs surrounding water and swells in a certain temperature range, thereby increasing the viscosity. Such a phenomenon is referred to as "gelatinization" of starch. Starch is widely used as a thickener or a shape retainer for processed foods because of its gelatinizing property. However, in the native unmodified starch, starch granules are collapsed at the time of gelatinization or by subsequent heating or stirring, and the initial viscosity cannot be maintained. This is a so-called "breakdown". When breakdown occurs, not only the viscosity decreases, but also amylose and amylopectin eluted from starch granules crystallize over time (retrogradation). As a result, the quality of starch gradually deteriorates.

One of means for suppressing breakdown of starch is to physically or chemically modify starch. Chemical modification of starch is a means for chemically modifying glucose constituting starch. On the other hand, physical modification is a means for changing physical properties of starch mainly by physical treatment such as heating. Physical modification is advantageous in that it is less likely to generate new chemical materials as by chemical modification and is easily used for a food material.

The physical modification may be performed by adding a substance other than starch. For example, attempts have been made to physically modify starch by adding various water-soluble polysaccharides. In Patent Document 1, xanthan gum and starch are powder-mixed, and then water is added to adjust the moisture content. Then, the obtained mixture is heat-treated at 100°C to 200°C for 30 minutes to 5 hours under dry conditions to modify starch. In addition, Patent Document 2 discloses a starch modifying means including subjecting a mixture of starch and a fruit-derived dietary fiber to a moist-heat treatment.

PRIOR ART DOCUMENT

PATENT DOCUMENTS

Patent Document 1: JP-A-2005-54028
Patent Document 2: WO 2018/216748 A

SUMMARY OF THE INVENTION

PROBLEM TO BE SOLVED BY THE INVENTION

However, although the modified starch obtained by the above methods favorably exhibits viscosity by heating, there is still room for improvement in viscosity decrease by high temperature heating such as retort sterilization conditions or temporal stability of viscosity.

Therefore, the present invention relates to providing a modified starch that exhibits favorable viscosity by heating and is less likely to decrease in viscosity even by high temperature heating, or has excellent viscosity stability after gelatinization.

MEANS FOR SOLVING THE PROBLEM

As a result of intensive studies, the present inventors prepared a mixture for heat treatment containing starch and a plant-derived dietary fiber or a mushroom-derived dietary fiber and having a specific moisture content. Then, under the condition that the pH at 25°C when 5 g of the mixture for heat treatment was suspended in 95 ml of water was 5 to 12, the mixture for heat treatment was subjected to dry heat treatment to obtain a physically modified starch. Then, the present inventors have found that the physically modified starch hardly decreases in viscosity even when heated at a high temperature such as a retort sterilization treatment, and is excellent in stability of viscosity after gelatinization, leading to the present invention.

That is, the present invention provides the following method for producing a physically modified starch.

[1] A method for producing a physically modified starch, comprising subjecting a mixture for heat treatment to dry heat treatment,

wherein the mixture for heat treatment comprises (A) starch, (B) a plant-derived dietary fiber or a mushroom-derived dietary fiber, and 0 to 20 mass% of moisture, and
a pH at 25°C when 5 g of the mixture for heat treatment is suspended in 95 ml of water is 5 to 12.

[2] The production method according to [1], wherein the dry heat treatment is performed at a temperature of 100 to 200°C.
[3] The production method according to [1] or [2], wherein the mixture for heat treatment further comprises (C) a basic compound.
[4] The production method according to [3], further comprising preparing an intermediate mixture comprising (A) the starch, (B) the plant-derived dietary fiber or the mushroom-derived dietary fiber, (C) the basic compound, and 20 to 99 mass% of moisture, and drying the intermediate mixture.
[5] The production method according to [3], further comprising allowing (B) the plant-derived dietary fiber or the mushroom-derived dietary fiber to coexist in the mixture for heat treatment without adding water.
[6] The production method according to any one of [1] to [5], wherein (A) the starch comprises one kind or two or more kinds selected from the group consisting of corn starch, potato starch, tapioca starch, rice starch, wheat starch, sweet potato starch, mung bean starch, arrowroot starch, and sago starch, and waxy starch thereof.
[7] The production method according to any one of [1] to [6], wherein (B) the plant-derived dietary fiber is one kind or two or more kinds selected from the group consisting of a citrus-derived dietary fiber, an apple-derived dietary fiber, an orange-derived dietary fiber, a pea-derived dietary fiber, a soybean-derived dietary fiber, a carrot-derived dietary fiber, a tomato-derived dietary fiber, a bamboo-derived dietary fiber, a sugar beet-derived dietary fiber, a seed coat-derived dietary fiber, a corn-derived dietary fiber, a wheat-derived dietary fiber, a barley-derived dietary fiber, a rye-derived dietary fiber, and a konjac-derived dietary fiber.
[8] The production method according to any one of [1] to [7], wherein (C) the basic compound is one kind or two or more kinds selected from the group consisting of hydroxides, carbonates, hydrogencarbonates, and organic acid salts of alkali metals, and hydroxides, carbonates, hydrogencarbonates, and organic acid salts of alkaline earth metals.
[9] The production method according to any one of [1] to [8], wherein a content of the component (B) with respect to 100 parts by mass of the component (A) in the mixture for heat treatment is 0.5 parts by mass or more and less than 30 parts by mass.
In addition, the present invention provides the following method for suppressing viscosity decrease at the time of gelatinization of starch or by subsequent high temperature heating, or imparting viscosity stability after gelatinization.
[10] A method for suppressing viscosity decrease at a time of gelatinization of starch or by subsequent high temperature heating, or imparting viscosity stability after gelatinization, comprising allowing an effective amount of a plant-derived dietary fiber or a mushroom-derived dietary fiber to coexist with starch and subjecting a mixture to dry heat treatment.
Furthermore, the present invention provides an agent for the following physical starch modification.
[11] An agent for physically modifying starch by dry heat treatment, comprising an effective amount of a plant-derived dietary fiber or a mushroom-derived dietary fiber.
Furthermore, the present invention provides the following physically modified starch, and a formulation and food or beverage product using the same.
[12] A physically modified starch comprising a plant-derived dietary fiber or a mushroom-derived dietary fiber, wherein when a viscosity of a water suspension of 4.8 mass% of the starch is measured under first conditions using a rapid viscoanalyzer, a difference between a maximum value of viscosity Va during a period from a gelatinization period to a high temperature holding period and a minimum value of viscosity Vb during a cooling period, that is, Va-Vb, is 100 mPa·s or less,
the first conditions are that

(1) a sample temperature is held at 50°C for a hydration period of 0 to 60 seconds,

raised at 0.203°C/sec for the gelatinization period of 60 to 282 seconds,
held at 95°C for the high temperature holding period of 282 to 432 seconds,
lowered at 0.197°C/sec for the cooling period of 432 to 660 seconds, and

held at 50°C for a cool holding period of 660 to 780 seconds, and

(2) a rotating speed of a paddle is 960 rpm from 0 to 10 seconds, and 160 rpm after 10 seconds.

[13] The physically modified starch according to [12], wherein an initial viscosity increase rate (Vr) is 0.02/s or less when the viscosity of the water suspension of 4.8 mass% of the starch is measured under the first conditions using the rapid viscoanalyzer.

[14] A physically modified starch comprising a plant-derived dietary fiber or a mushroom-derived dietary fiber, wherein when a viscosity of a water suspension of 4.8 mass% of the starch is measured under second conditions using a rapid viscoanalyzer, a difference between a maximum value of viscosity Va during a period from a gelatinization period to a high temperature holding period and a minimum value of viscosity Vb during a cooling period, that is, Va-Vb, is 150 mPa·s or less,
the second conditions are that

(1) a sample temperature is held at 50°C for a hydration period of 0 to 60 seconds,

raised at 0.194°C/sec for the gelatinization period of 60 to 420 seconds,
held at 120°C for the high temperature holding period of 420 to 1020 seconds, and
lowered at 0.194°C/sec for the cooling period of 1020 to 1380 seconds, and

(2) a rotating speed of a paddle is 960 rpm from 0 to 10 seconds, and 160 rpm after 10 seconds.

[15] The physically modified starch according to [14], wherein an initial viscosity increase rate (Vr) is 0.02/s or less when the viscosity of the water suspension of 4.8 mass% of the starch is measured under the second conditions using the rapid viscoanalyzer.

[16] A physically modified starch comprising a plant-derived dietary fiber or a mushroom-derived dietary fiber,

wherein a relative value A of viscosities before and after retort sterilization treatment defined below is 80% or more and a relative value B of viscosities during storage after the retort sterilization treatment is 85% to 125% in a viscosity of a gelatinized starch paste obtained from a water suspension containing 5 mass% of the starch:

$$\text{relative value A of viscosities before and after retort sterilization treatment (\%)} = \eta_2/\eta_1 \times 100,$$

$$\text{relative value B of viscosities during storage after retort sterilization treatment (\%)} = \eta_3/\eta_2 \times 100;$$

wherein
the water suspension containing 5 mass% of the physically modified starch is heated in a hot water bath while being stirring, kept at 90°C for 10 minutes after reaching 90°C, and then cooled in a constant temperature water bath at 20°C for 1 hour, and then a viscosity measured at 60 rpm using a B-type rotational viscometer is defined as $\eta_1$,
the water suspension containing 5 mass% of the physically modified starch is heated in a hot water bath while being stirred, kept at 90°C for 10 minutes after reaching 90°C, further subjected to a retort sterilization treatment under condition of 121°C for 20 minutes, allowed to cool at 20°C for 1 day, and then a viscosity measured at 60 rpm using a B-type rotational viscometer is defined as $\eta_2$,
the water suspension containing 5 mass% of the physically modified starch is heated in a hot water bath while being stirred, kept at 90°C for 10 minutes after reaching 90°C, further subjected to a retort sterilization treatment under conditions of 121°C for 20 minutes, allowed to cool at 20°C for 1 day, then stored in a constant temperature chamber at 5°C for 1 week (7 days), and temperature-controlled by being immersed in a constant temperature water bath at 20°C for 1 hour, and then a viscosity measured at 60 rpm using a B-type rotational viscometer is defined as $\eta_3$.

[17] A formulation comprising the physically modified starch according to any one of [12] to [16].

[18] A food or beverage product comprising the physically modified starch according to any one of [12] to [16].

[19] A formulation comprising a physically modified starch produced by the production method according to any one of [1] to [9].

[20] A food or beverage product comprising a physically modified starch produced by the production method according to any one of [1] to [9].

## EFFECT OF THE INVENTION

According to the configuration of the present invention, it is possible to obtain a modified starch in which breakdown of starch during heating is suppressed, viscosity expression by heating is good, and a temporal change in viscosity after gelatinization is suppressed.

## BRIEF DESCRIPTION OF THE DRAWINGS

Fig. 1A is a conceptual diagram illustrating a typical RVA viscosity curve, and Va and Vb. When a peak is observed as in the RVA viscosity curve of the solid line, Va is the viscosity ($Va_i$) at the peak. When there is a minimum value at the time of temperature fall, the value is Vb ($Vb_i$). As in the RVA viscosity curve of the broken line, there is no peak or minimum value, and when the viscosity changes monotonically, Va and Vb coincide ($Va_j$ and $Vb_j$). Fig. 1B is a conceptual diagram showing a method for calculating an initial viscosity increase rate (Vr).

Fig. 2 is an RVA viscosity curve obtained by measuring the viscosity of each starch sample by RVA (Profile 1, maximum temperature 95°C) in Test Example 1.

Fig. 3 is an RVA viscosity curve obtained by measuring the viscosity of each starch sample by RVA (Profile 2, maximum temperature 120°C) in Test Example 1.

Fig. 4 shows photographs obtained by observing each gelatinized starch paste with an optical microscope after evaluation using RVA in profiles 1 and 2 (maximum temperature 95°C, 120°C) in Test Example 1.

## MODE FOR CARRYING OUT THE INVENTION

Symbols and abbreviations in the present specification are understood, unless otherwise limited, in meanings commonly used in the technical field to which the present invention belongs, according to the context of the present specification.

In the present specification, the phrase "containing" is used for the purpose of including the phrase "consisting essentially of" and the phrase "consisting of".

In the present specification, the term "starch" refers to a starch (so-called raw starch) that has not been denatured by physical treatment or chemical treatment. That is, the starch is obtained by breaking the cell wall, taking out and collecting starch granules in the plant, and is not subjected to any processing such as chemical treatment.

The "physically modified starch" in the present specification refers to a starch in which the properties of swelling and/or collapse of starch granules of a raw starch by physical stimulation such as exposure to water, heating and/or stirring by physical treatment such as heating and pressurization are decreased.

In the present specification, as long as a functional group is not chemically added or introduced, even when a treatment is performed with an acid, an alkali, or the like simultaneously with a physical treatment, it is considered that the compound is physically modified.

On the other hand, in the present specification, a "chemically modified starch" is a starch in which a functional group is added to or introduced into a hydroxyl group of a constituent glucose unit, and is distinguished from a physically modified starch.

For example, in Japan, the United States, and Europe, the physically modified starch is classified as common foods, and the chemically modified starch is classified as a "chemically modified starch" which is a food additive.

In the present specification, "gelatinization" and "gelatinization" are not particularly distinguished, and both indicate that starch granules are supplied with water to be in a swollen or collapsed state. At this state, water is entering between amylopectin chains of starch granules.

Unless otherwise limited, the steps, processes, or operations described herein may be performed at room temperature.

In the present specification, room temperature means a temperature within a range of 10 to 40°C. In addition, in the present specification, heating means a treatment for making the temperature of the mixture having a temperature equal to or lower than room temperature higher than that before the treatment.

As used herein, "substantially free" means that it, if present, is at a level below the limit of detection, for example, less than 0.1%.

[Method for producing physically modified starch]

The method for producing a physically modified starch of the present invention includes subjecting a mixture for heat treatment containing (A) starch, (B) a plant-derived dietary fiber or a mushroom-derived dietary fiber, and a predetermined amount of moisture to dry heat treatment. Here, when 5 g of the mixture for heat treatment is suspended in 95 ml of water, the pH at 25°C is 5 to 12.

(Component (A): Starch)

The starch as the component (A) used in the present invention is not particularly limited, but is preferably one kind or a combination of two or more kinds selected from the group consisting of corn starch, potato starch, tapioca starch, rice starch, wheat starch, sweet potato starch, mung bean starch, arrowroot starch and sago starch, and waxy starch (e.g., waxy potato starch, waxy rice starch, waxy corn starch) thereof, and more preferably one kind alone or a combination of two or more kinds selected from the group consisting of potato starch, tapioca starch, rice starch, corn starch and sweet potato starch, and waxy starch thereof.

In one embodiment, the starch as the component (A) is one kind or two or more kinds selected from waxy corn starch, tapioca starch, and potato starch. When the modification is performed using these starches, the obtained modified starch is preferable from the viewpoint of suppressing a decrease in viscosity due to a shear such as stirring and excellent viscosity stability.

The particle size (longest diameter) of the starch granules is, for example, 15 to 100 μm in the case of potato starch and its waxy starch, for example, 5 to 35 μm in the case of tapioca starch and its waxy starch, for example, 2 to 10 μm in the case of rice starch and its waxy starch, for example, 6 to 25 μm in the case of corn starch and its waxy starch, for example, 10 to 35 μm in the case of wheat starch, for example, 15 to 35 μm in the case of sweet potato starch, for example, 15 to 25 μm in the case of mung bean starch, for example, 3 to 15 μm in the case of arrowroot starch, and for example, 10 to 60 μm in the case of sago starch.

The standard deviation of the particle size (longest diameter) of the starch granules is, for example, 1 to 60 μm in the case of potato starch and its waxy starch, for example, 1 to 15 μm in the case of tapioca starch and its waxy starch, for example, 0.1 to 5 μm in the case of rice starch and its waxy starch, for example, 1 to 11 μm in the case of corn starch and its waxy starch, for example, 1 to 15 μm in the case of wheat starch, for example, 1 to 15 μm in the case of sweet potato starch, for example, 0.1 to 5 μm in the case of mung bean starch, for example, 0.1 to 5 μm in the case of arrowroot starch, and for example, 1 to 30 μm in the case of sago starch.

The ratio of the longest diameter/shortest diameter of the starch granules can be, for example, in the range of about 1.0 to about 1.5, in the range of about 1.1 to about 1.5, or about 1.0.

(Component (B): plant-derived dietary fiber or mushroom-derived dietary fiber)

Examples of the component (B) of the present invention include any one of a plant-derived dietary fiber and a mushroom-derived dietary fiber, or a combination of both of them.

As the plant-derived dietary fiber used as the component (B) of the present invention, a plant-derived dietary fiber that is generally distributed can be widely used. The plant-derived dietary fiber used in the present invention may be a residue obtained by removing a fruit juice or a vegetable juice after squeezing fruit or vegetables, or a purified product thereof. These plant-derived dietary fibers can be produced by a known method and/or commercially obtained.

The plant-derived dietary fiber used in the present invention may be a water-soluble dietary fiber or a water-insoluble dietary fiber, but is preferably a complex-type dietary fiber containing the water-soluble dietary fiber and the water-insoluble dietary fiber. Examples of the water-soluble dietary fiber include one kind alone or a combination of two or more kinds selected from the group consisting of water-soluble hemicellulose, pectin, β-glucan, glucomannan, xylan, galacturonan, galactomannan, and xyloglucan. More specifically, examples of the water-soluble dietary fiber include one kind alone or a combination of two or more kinds selected from the group consisting of guar gum, locust bean gum, tamarind seed gum, psyllium seed gum, agar, carrageenan, arabic gum and ghatti gum. Examples of the insoluble dietary fiber include one kind alone or a combination of two or more kinds selected from the group consisting of cellulose, lignin, insoluble hemicellulose, and protopectin.

The complex-type dietary fiber can preferably contain a dietary fiber in an amount of 40 mass% or more, more preferably 50 mass% or more. In some cases, the complex-type dietary fiber may contain a dietary fiber in an amount of 60 mass% or more or 70 mass% or more. The complex-type dietary fiber preferably contains insoluble dietary fiber in an amount of 20 mass% or more, more preferably about 30 mass%. In some cases, the complex-type dietary fiber can contain 40 mass% or more, 50 mass% or more, or 60 mass% or more of the insoluble dietary fiber. The complex-type dietary fiber preferably contains, for example, 10 mass% or more, 20 mass% or more, 30 mass% or more, 40 mass% or more, or 50 mass% or more of the water-soluble dietary fiber. The mass ratio of the water-insoluble dietary fiber and the water-soluble dietary fiber contained in the complex-type dietary fiber can be preferably in the range of 1 : 0.001 to 1 : 15, more preferably 1 : 0.01 to 1 : 15, further preferably 1 : 0.2 to 1 : 15, and further more preferably 1 : 0.3 to 1 : 10.

In the present invention, the dietary fiber content, the water-soluble dietary fiber content, and the insoluble dietary fiber content are measured by the modified Prosky method.

In one embodiment, the plant-derived dietary fiber may have a plant tissue structure. The dietary fiber having a plant tissue structure refers to a dietary fiber in which a cell wall structure of a plant remains. The presence or absence of the cell wall structure can be determined by, for example, whether or not a structure corresponding to the cell wall structure is observed when the dietary fiber or the content thereof is observed with a microscope.

The plant-derived dietary fiber may preferably be a dietary fiber containing pectin.

In one embodiment, as the plant-derived dietary fiber, one kind alone or a combination of two or more kinds thereof can be used from fruit-derived dietary fibers below, and legume-derived dietary fibers such as a pea-derived dietary fiber and a soybean-derived dietary fiber, a carrot-derived dietary fiber, vegetable-derived dietary fibers such as a tomato-derived dietary fiber, a bamboo-derived dietary fiber, a sugar beet-derived dietary fiber, a seed coat-derived dietary fiber (a basil seed-derived dietary fiber, a chia seed-derived dietary fiber, etc.), a corn-derived dietary fiber, a wheat-derived dietary fiber, a barley-derived dietary fiber, a rye-derived dietary fiber, a konjac-derived dietary fiber, and the like.

As the plant-derived dietary fiber used in the present invention, one kind or two or more kinds selected from the group consisting of a fruit-derived dietary fiber, a seed coat-derived dietary fiber, and a legume-derived dietary fiber are preferable, the fruit-derived dietary fiber is more preferable, one kind or two or more kinds selected from the group consisting of a citrus-derived dietary fiber (citrus fiber), an apple-derived dietary fiber, and an orange-derived dietary fiber are further preferable, and a citrus-derived dietary fiber is particularly suitably used.

The fruit-derived dietary fiber is a composition obtained by dry-pulverization a fraction containing dietary fiber of fruit. The fruit-derived dietary fiber is characterized in that it contains many insoluble dietary fibers such as cellulose and hemicellulose in the cell walls of cells constituting the fruit, and partially retain the fiber tissue structure of the fruit. The fruit-derived dietary fiber can be, for example, one in which the micelle structure of the cell is broken and has a porous structure.

The origin of the fruit-derived dietary fiber used in the present invention is not particularly limited as long as it exerts the effects of the present invention, and examples thereof include citrus fruits (a lemon, a lime, a yuzu, an orange, a grapefruit, a Chinese citron, a Satsuma mandarin, a Citrus sudachi, a Citrus sphaerocarpa, a Citrus hassaku, a Citrus iyo (iyokan), a Citrus maxima, a Citrus grandis, a kumquat, a Citrus aurantium, etc.), an apple, a pineapple, a banana, a guava, a mango, an acerola, a papaya, a passion fruit, a peach, grape (contains a muscat grape, a kyoho grape, and the like), a strawberry, a Japanese plum, a pear, an apricot, a plum, a kiwi fruit, a melon, a persimmon, and a fig. Among them, a dietary fiber derived from the citrus fruits are preferable, and a dietary fiber derived from one kind or two or more kinds selected from the group consisting of a lemon, a lime, a yuzu, an orange, a grapefruit, and a Chinese citron are more preferable.

The fruit-derived dietary fiber used in the present invention is preferably prepared from a residue obtained by removing fruit juice from the fruit or a purified product obtained by purifying the residue by filtration or the like.

The residue (mesh-on) when the powder of the fruit-derived dietary fiber is sieved through a 60 mesh sieve is preferably 3.0 mass% or less, more preferably 2.5 mass% or less, and further preferably 2.0 mass% or less with respect to the total amount of the powder subjected to the sieving.

The water holding capacity after immersing 1 part by mass of the fruit-derived dietary fiber in water at 25°C for 24 hours is preferably 5 parts by mass or more, more preferably 10 parts by mass or more, further preferably 15 parts by mass or more, and particularly preferably 20 parts by mass or more. The water holding capacity is a mass obtained by subtracting a mass before being immersed in water from a mass after the dietary fiber immersed in water is centrifuged to remove water.

The dietary fiber content of the fruit-derived dietary fiber is preferably 85 mass% or more.

The seed coat-derived dietary fiber can be obtained, for example, by adding water to the seed to swell the polysaccharide component of the seed coat (outer shell), and then applying physical force by stirring or the like to remove the seed portion. As a more specific method for preparing the seed coat-derived dietary fiber, for example, a method similar to the method described in WO 2016/114201 A can be used.

The mushroom-derived dietary fiber used as the component (B) of the present invention is not particularly limited as long as it is a dietary fiber obtained from mushroom. Herein, the mushroom represents, for example, a fruiting body of Basidiomycota or Ascomycota. These mushroom-derived dietary fibers can be produced by a known method and/or commercially obtained.

In one embodiment, the plant-derived dietary fiber used in the present invention may be a mushroom water extract.

The mushroom-derived dietary fiber used in the present invention may be a water-soluble dietary fiber or a water-insoluble dietary fiber. Examples of the water-soluble dietary fiber include an acidic heteropolysaccharide containing glucuronic acid. Examples of the water-insoluble dietary fiber include cellulose, lignin, insoluble hemicellulose, protopectin, and chitin.

As the mushroom-derived dietary fiber of the component (B), a generally distributed mushroom-derived dietary fiber can be widely used. Specific examples of the mushroom-derived dietary fiber include white jelly fungus-derived dietary fiber such as white jelly fungus extract. More specific embodiments and production methods of the white jelly fungus-derived dietary fiber can be understood from, for example, JP 2019-195308 A and JP 2019-041735 A.

(pH of mixture for heat treatment)

When 5 g of the mixture for heat treatment is suspended in 95 ml of water, the pH at 25°C is 12 or less, and may be, for example, 11.5 or less, 11 or less, 10.5 or less, 10 or less, or 8 or less, from the viewpoint of facilitating the preparation of the mixture for heat treatment. When 5 g of the mixture for heat treatment is suspended in 95 ml of water, the pH at 25°C is 5 or more, for example, 5.5 or more from the viewpoint of remarkably exhibiting the effects of the present invention.

(Component (C): basic compound)

In the production method of the present invention, although not particularly limited, it is preferable that the mixture for heat treatment further contains a basic compound which is the component (C). The basic compound is not particularly limited as long as it can be used for food, and is, for example, one kind or two or more kinds selected from the group consisting of a hydroxide, a carbonate, a hydrogencarbonate, and an organic acid salt of an alkali metal, and a hydroxide, a carbonate, a hydrogencarbonate, and an organic acid salt of an alkaline earth metal. Examples of the organic acid include a citric acid, a tartaric acid, a malic acid, a succinic acid, a gluconic acid, a fumaric acid, an acetic acid, and an oxalic acid.

Among them, as the basic compound, a carbonate or a hydrogencarbonate of an alkali metal is preferable, and the carbonate of an alkali metal is more preferable.

(Ratio of each component)

The total content of the component (B) with respect to 100 parts by mass of the total content of the component (A) in the mixture for heat treatment is preferably 0.5 parts by mass or more, more preferably 1 part by mass or more, and may be, for example, 2 parts by mass or more, 3 parts by mass or more, 4 parts by mass or more, or 5 parts by mass or more, from the viewpoint of remarkably exhibiting the effects of the present invention.

The total content of the component (B) with respect to 100 parts by mass of the total content of the component (A) in the mixture for heat treatment is preferably less than 30 parts by mass, and may be, for example, 20 parts by mass or less, for example, 15 parts by mass or less, 10 parts by mass or less, or 8 parts by mass or less. Such a content of the component (B) is preferable from the viewpoint that the mixture for heat treatment can be easily prepared and being colored during heating is suppressed.

The total content of the component (C) with respect to 100 parts by mass of the total content of the component (A) in the mixture for heat treatment can be appropriately adjusted based on the pH of the raw starch as a raw material, and is, for example, 0.001 parts by mass or more, 0.01 parts by mass or more, 0.03 parts by mass or more, or 0.05 parts by mass or more.

The total content of the component (C) with respect to 100 parts by mass of the total content of the component (A) in the mixture for heat treatment can be appropriately adjusted based on the pH of the raw starch as a raw material, and is, for example, 10 parts by mass or less, 5 parts by mass or less, or 1 part by mass or less.

(Moisture, pH, and other components)

The moisture content of the mixture for heat treatment is 0 to 20 mass%, preferably 0 to 15 mass%, and more preferably 0 to 14.9 mass%, and it is more preferable that the mixture for heat treatment does not substantially contain moisture.

The mixture for heat treatment may be used, depending on the intended use of the obtained modified starch, in addition to the components (A) to (C) and moisture, as other components, one kind or two or more kinds selected from the group consisting of a water-soluble polysaccharide; a water-insoluble polysaccharide; an excipient; an emulsifier; a known nutritional component that can be used in a food, a pharmaceutical product, a quasi-pharmaceutical product, or a cosmetic product; an alcohol; an alcoholic liquor; salts; a taste component; a fruit juice; a flesh of fruit; vegetables; a vegetable juice; a puree; an extract (derived from an animal, a plant, a microorganism, etc.); a spice; a sweetener; a sugar alcohol; a high-intensity sweetener; a bittering agent; a flavor; a food coloring; and other food additives (a preservative, an antioxidant, a thickener, a stabilizers, etc.); an active ingredient or additive for a pharmaceutical a quasi-pharmaceutical product, or a cosmetic product; other antiseptics; an antifungal agent; a surfactant; a gelling agent; a solvent; a fragrance, ;a bactericide; and a deodorizing component may be contained.

The water-soluble polysaccharide as another component is not particularly limited, and examples thereof include a xanthan gum or a gellan gum. The water-insoluble polysaccharide is not particularly limited, and examples thereof include cellulose or hemicellulose.

(Dry heat treatment)

The "dry heat treatment" is a treatment in which heating is performed without adding moisture from the outside during the heating step. The dry heat treatment is not particularly limited, but for example, a convection heat transfer drying device or a conduction heat transfer drying device can be used. Among them, it is preferable to use a mixer type heating device or the like provided with a jacket that can simultaneously perform mixing and heating, can increase the residence time, and can heat the surroundings from the viewpoint of improving the treatment efficiency.

The temperature of the dry heat treatment is, for example, 100°C or higher, preferably 120°C or higher, and more preferably 150°C or higher from the viewpoint of remarkably exhibiting the effects of the present invention.

The temperature of the dry heat treatment is, for example, 200°C or lower, preferably 190°C or lower, and more preferably 180°C or lower from the viewpoint of preventing or suppressing the coloring of the starch.

The time for the dry heat treatment can be appropriately set depending on the type of starch and a plant-derived dietary fiber or a mushroom-derived dietary fiber, and the dry heat treatment temperature, but is 10 minutes or more, preferably 20 minutes or more, and more preferably 30 minutes or more from the viewpoint of remarkably exhibiting the effects of the present invention.

The time for the dry heat treatment can be appropriately set depending on the types of starch and a plant-derived dietary fiber and the dry heat treatment temperature, but is 1440 minutes or less, preferably 1200 minutes or less, more preferably 720 minutes or less, further preferably 540 minutes or less, and for example, 240 minutes or less from the viewpoint of preventing being colored.

(Other steps)

When the mixture for heat treatment contains the component (A) and the component (B), and optionally the component (C), it is not particularly limited, but for example, it is preferable to include the following step (i) or (ii), but it is more preferable to include the step (i) from the viewpoint of remarkably exhibiting the effects of the present invention.

(i) Preparing an intermediate mixture containing (A) starch, (B) a plant-derived dietary fiber or a mushroom-derived dietary fiber and 20 to 99 mass% of moisture, and optionally (C) a basic compound; and
drying the intermediate mixture.
(ii) Adding (B) a plant-derived dietary fiber or a mushroom-derived dietary fiber to a mixture including (A) starch and 0 to 20 mass% of moisture, and optionally (C) a basic compound without adding water.

Here, in the (ii), "without adding water" means that water is not added to the component (B) itself and the mixture obtained by adding the component (B).

When the step of (ii) is included, it is preferable that the production method of the present invention further includes the step of (iii) preparing an intermediate mixture containing (A) starch and 20 to 99 mass% of moisture, and optionally (C) a basic compound, and drying the intermediate mixture until a moisture content of 0 to 20 mass% is attained.

The drying in the steps (i) to (iii) is not particularly limited, but for example, freeze drying, natural drying, or drying in a constant temperature chamber or the like can be used. The drying temperature is, for example, -50 to 70°C.

The intermediate mixture obtained by drying is preferably further pulverized in order to uniformly perform dry heat treatment. For the pulverization, a hammer mill, a wing mill, a ball mill, a jet mill, a mortar, a mill-type pulverizer, or the like is used.

The intermediate mixture obtained by drying may be further pH adjusted. The pH adjustment is performed, for example, by adding a predetermined amount of a basic substance to the obtained intermediate mixture. Examples of the basic substance include a substance that exhibits alkalinity (for example, a pH of 8 or more) when dissolved in water. Specific examples thereof include a hydroxide, a carbonate, a hydrogencarbonate, and an organic acid salt of a metal of Group 1 of the periodic table (e.g., sodium, potassium) ; and a hydroxide, a carbonate, a hydrogencarbonate, or an organic acid salt of a metal of Group 2 of the periodic table (e.g., calcium, magnesium).

The intermediate mixture obtained by drying is preferably further subjected to humidity control, sieving, and the like.

The production method of the present invention may further include one or more post-treatment steps selected from the group consisting of pulverization of a physically modified starch, humidity control, and sieving.

The production method of the present invention may further include a step of washing a mixture for heat treatment containing starch or a physically modified starch with water or an organic solvent (ethanol or the like) before or after the dry heat treatment.

(Specific Method 1: dry heat treatment of powder containing starch and plant-derived dietary fiber or mushroom-derived dietary fiber and optionally basic compound)

One embodiment of the production method of the present invention includes a method of preparing a mixture containing (A) starch, (B) a plant-derived dietary fiber or a mushroom-derived dietary fiber, and optionally (C) a basic compound, drying the obtained mixture, and then subjecting the mixture to dry heat treatment.

In the method, first, a plant-derived dietary fiber or a mushroom-derived dietary fiber and optionally water are added to starch, and further optionally a basic compound is mixed so as to be uniform, thereby preparing an intermediate mixture having a moisture content of 20 to 99 mass%. At this time, the order of addition of starch, water, the plant-derived dietary fiber or the mushroom-derived dietary fiber, or the basic compound is not particularly limited. When the basic compound is added, from the viewpoint of avoiding a local increase in pH of starch, it is preferable that the basic compound is added to a mixture of starch, water, and the plant-derived dietary fiber or the mushroom-derived dietary fiber as it is or in a state of being dispersed in water, and the mixture is mixed. The mixing may be performed by a known method such as stirring.

The total content of the component (B) with respect to 100 parts by mass of the total content of the component (A) in the intermediate mixture is preferably 0.5 parts by mass or more and less than 30 parts by mass, more preferably 0.5 to 20 parts by mass, further preferably 1 to 15 parts by mass, further more preferably 1 to 10 parts by mass, and for example, 1 to 8 parts by mass.

The total content of the component (C) with respect to 100 parts by mass of the total content of the component (A) in the intermediate mixture is preferably 0.001 to 10 parts by mass, more preferably 0.01 to 5 parts by mass, further preferably 0.03 to 1 part by mass, and for example, 0.05 to 1 part by mass.

The obtained intermediate mixture is dried after further adding the above-mentioned other components as necessary. The moisture content of the dried product obtained by this is, for example, 0 to 20 mass%, preferably 0 to 15 mass%, and more preferably 0 to 14.9 mass%.

The intermediate mixture before the drying is preferably pH adjusted. The pH is adjusted to, for example, 5 to 12.

Furthermore, the dried product is preferably pulverized from the viewpoint of uniformly performing dry heat treatment. The means for pulverization is the same as that of the above mixture for heat treatment.

The dried product may be directly subjected to dry heat treatment as a mixture for heat treatment, or may be further subjected to a pulverization step or a step of adding the other components described above to obtain a mixture for heat treatment.

The above-mentioned other components may be added at the preparation stage of the intermediate mixture or the dried product as long as the effects of the present invention are not impaired, or may be added to the modified starch obtained after the dry heat treatment from the viewpoint of suppressing the denaturation of the components by the dry heat treatment.

(Specific Method 2: dry heat treatment of mixture of powder containing starch and optionally basic compound and plant-derived dietary fiber powder or mushroom-derived dietary fiber powder)

One embodiment of the method for producing a physically modified starch of the present invention includes an aspect in which an intermediate mixture containing (A) starch and optionally a basic compound (C) is prepared and dried, and further (B) a plant-derived dietary fiber or a mushroom-derived dietary fiber is added to the dried intermediate mixture and the intermediate mixture is subjected to dry heat treatment.

In the method, first, water and a basic compound are optionally added to starch so that the starch and the basic compound are uniformly mixed, and an intermediate mixture is prepared so that the moisture content is 20 to 99 mass%. At this time, the order of addition of starch, water, or the basic compound is not particularly limited. When the basic compound is added, it is preferable to add the basic compound to a mixture of starch and water and mix them from the viewpoint of avoiding a local pH increase. The mixing may be performed by a known method such as stirring.

The preferred range of the total content of the component (C) with respect to 100 parts by mass of the total content of the component (A) in the intermediate mixture is the same as in the case of the Specific Method 1.

The obtained intermediate mixture is dried in the same manner as in the Specific Method 1 to obtain a dried product. In addition, similarly to the Specific Method 1, a pH adjustment step and a pulverization step may be included as necessary during the drying.

To the obtained dried product, a plant-derived dietary fiber or a mushroom-derived dietary fiber is added as the component (B). At this time, the moisture content of the plant-derived dietary fiber or the mushroom-derived dietary fiber is, for example, 0 to 20 mass%, preferably 0 to 15 mass%, more preferably 0 to 14.9 mass%, from the viewpoint of remarkably exhibiting the effects of the present invention, and it is more preferable to be substantially free of moisture.

Based on 100 parts by mass of the total content of the component (A) in the dried product, the amount of the plant-derived dietary fiber or the mushroom-derived dietary fiber to be added is preferably 0.5 parts by mass or more and less than 30 parts by mass, more preferably 0.5 to 20 parts by mass, further preferably 1 to 15 parts by mass, and further more preferably 1 to 10 parts by mass, for example, 1 to 8 parts by mass.

The mixture containing the components (A) to (C) obtained by adding the plant-derived dietary fiber or the mushroom-derived dietary fiber to the dried product may be directly subjected to dry heat treatment as a mixture for heat treatment, or may be further subjected to a pulverization step or a step of adding the other components described above to obtain a mixture for heat treatment.

The above-mentioned other components may be added at the preparation stage of the intermediate mixture or the dried product as long as the effects of the present invention are not impaired, or may be added to the modified starch obtained after the dry heat treatment from the viewpoint of suppressing the denaturation of the components by the dry heat treatment.

[Method for imparting viscosity stability after gelatinization to starch]

The method of the present invention is a method for suppressing viscosity decrease at the time of gelatinization of starch or by subsequent high temperature heating or imparting viscosity stability after gelatinization, and is characterized in that an effective amount of a plant-derived dietary fiber or a mushroom-derived dietary fiber is allowed to coexist with starch and subjected to dry heat treatment.

The moisture content in a state in which an effective amount of the plant-derived dietary fiber or the mushroom-derived dietary fiber coexists with the starch is preferably 0 to 20 mass%, and more preferably 0 to 14.9 mass%.

Specific aspects of the plant-derived dietary fiber or the mushroom-derived dietary fiber, the starch, a basic compound, and the dry heat treatment and other steps are the same as those described in the section of [Method for producing physically modified starch].

[Use of plant-derived dietary fiber or mushroom-derived dietary fiber]

The present invention includes the use of a plant-derived dietary fiber or a mushroom-derived dietary fiber for the production of a modified starch by dry heat treatment. The production conditions and specific aspects of the plant-derived dietary fiber or the mushroom-derived dietary fiber related to the use are the same as those described in the section of [Method for producing physically modified starch].

[Agent for modifying physical starch]

The agent of the present invention is an agent for modifying physical starch by dry heat treatment, and contains an effective amount of a plant-derived dietary fiber or a mushroom-derived dietary fiber.

The agent according to the present invention may further contain, as components other than the plant-derived dietary fiber and the mushroom-derived dietary fiber, one kind or two or more kinds of an excipient; an emulsifier food; a known nutritional component that can be used in a food, a pharmaceutical product, a quasi-pharmaceutical product, or a cosmetic product; an alcohol; an alcoholic liquor; salts; a taste component; a fruit juice; a flesh of fruit; vegetables; a vegetable juice; a puree; an extract (derived from an animal, a plant, a microorganism, etc.); a spice; a sweetener; a sugar alcohol; a high-intensity sweetener; a bittering agent, a flavor, a food coloring, and other food additives (a preservative, an antioxidant, a thickener, a stabilizers, etc.); and an active ingredient or additive for a pharmaceutical; a quasi-pharmaceutical product, or a cosmetic product; other antiseptics; an antifungal agent; a surfactant; a gelling agent; a solvent; a fragrance; a bactericide; and a deodorizing component may be contained.

The dietary fiber is the same as that described in the section of [Method for producing physically modified starch]. In the use of the agent of the present invention, specific aspects of the target starch, the basic compound to be used, and the dry heat treatment and other steps can also be understood from the description of the same item.

[Physically Modified Starch]

The physically modified starch of the present invention (hereinafter, referred to as a modified starch of the present invention) contains at least a plant-derived dietary fiber or a mushroom-derived dietary fiber. The modified starch is not particularly limited, but for example, a modified starch produced by the above production method can be mentioned as a suitable example.

The modified starch of the present invention preferably has the following Characteristics (I) from the viewpoint of remarkably exhibiting the effects of the present invention.

Characteristics (I): The viscosity of a water suspension containing 4.8 mass% of the modified starch of the present invention was measured using a rapid viscoanalyzer under first conditions (Profile 1), and
the first conditions are that

(1) the sample temperature is held at 50°C for the hydration period of 0 to 60 seconds,

raised at 0.203°C/sec for the gelatinization period of 60 to 282 seconds,
held at 95°C for the high temperature holding period of 282 to 432 seconds,
lowered at 0.197°C/sec for the cooling period of 432 to 660 seconds, and
held at 50°C for the cool holding period of 660 to 780 seconds, and

(2) the rotating speed of the paddle is 960 rpm from 0 to 10 seconds, and 160 rpm after 10 seconds. At this time, the difference between the maximum value of viscosity Va during the period from the gelatinization period to the high temperature holding period and the minimum value of viscosity Vb during the cooling period, that is, Va-Vb, is 100 mPa·s or less, preferably 80 mPa·s or less, more preferably 50 mPa·s or less, and further preferably 35 mPa·s or less.

In addition, the modified starch of the present invention preferably has the following Characteristics (II) from the viewpoint of remarkably exhibiting the effects of the present invention.

Characteristics (II): The viscosity of a water suspension containing 4.8 mass% of the modified starch of the present invention is measured using a rapid viscoanalyzer under the first conditions (Profile 1). At this time, the initial viscosity increase rate is 0.02/s or less, more preferably 0.017/s or less, and further preferably 0.016/s or less.

Characteristics (III): The viscosity of a water suspension containing 4.8 mass% of the modified starch of the present invention was measured using a rapid viscoanalyzer under second conditions (Profile 2), and
the second conditions are that

(1) the sample temperature is held at 50°C for the hydration period from 0 to 60 seconds;

raised at 0.194°C/sec for the gelatinization period of 60 to 420 seconds,
held at 120°C for the high temperature holding period of 420 to 1020 seconds; and
lowered at 0.194°C/sec or the cooling period of 1020 to 1380 seconds, and

(2) the rotating speed of the paddle is 960 rpm from 0 to 10 seconds, and 160 rpm after 10 seconds. At this time, the difference between the maximum value of viscosity Va during the period from the gelatinization period to the high temperature holding period and the minimum value of viscosity Vb during the cooling period, that is, Va-Vb, is 150 mPa·s or less, more preferably 100 mPa·s or less, further preferably 80 mPa·s or less, and particularly preferably 50 mPa·s or less.

In addition, the modified starch of the present invention preferably has the following characteristics (IV) from the viewpoint of remarkably exhibiting the effects of the present invention.

Characteristics (IV): The viscosity of a water suspension containing 4.8 mass% of the modified starch of the present invention is measured using a rapid viscoanalyzer under the second condition (Profile 2). At this time, the initial viscosity increase rate is 0.02/s or less, more preferably 0.017/s or less, and further preferably 0.016/s or less.

The rapid viscoanalyzer (RVA) is a rotational viscometer capable of freely controlling temperature and setting rotation conditions optimized for measuring viscosity characteristics of starch, grain, flour, and the like. In the present specification, although not particularly limited, as the RVA, for example, one manufactured by NSP Perten is suitably used. In addition, examples of the similar measuring apparatus include an amyloviscograph (sometimes referred to as an amylograph or a viscograph) and a microviscograph manufactured by Brabender, a rheometer manufactured by Bohlin, a rotational viscometer manufactured by HAAKE, a rheometer manufactured by HAAKE, and a microvisco manufactured by HAAKE.

A change curve of viscosity with respect to time obtained when the above-described time-dependent temperature change is applied using the RVA is referred to as an RVA viscosity curve (see Fig. 1A). The RVA viscosity curve is divided into periods of hydration, gelatinization, high temperature holding, cooling, and cool holding, as shown in the two profiles.

In the present specification, two parameters, Va-Vb and the initial viscosity increase rate, are defined as follows. When these parameters are acquired, the measurement interval of the viscosity data of RVA is set to 4 seconds.

(a)Va-Vb

The maximum value of the viscosity during the period from the gelatinization period to the high temperature holding period in the RVA measurement condition is defined as Va, the minimum value of the viscosity during the cooling period is defined as Vb, and the value obtained by subtracting Vb from Va is defined as Va-Vb (unit: mPa·s).

(b)Initial viscosity increase rate (Vr)

In the gelatinization period under the RVA measurement condition, a graph is made in which the vertical axis represents relative viscosity $V_{Rel}$ when Va is 1, and the horizontal axis represents time (seconds). Then, a certain measurement point A on the graph is defined as a measurement point that satisfies the conditions that $V_{Rel}$ increases by 0.01 or more at any of the six consecutive measurement points immediately after A as compared with the measurement point immediately before the point A. At this time, linear approximation is performed on data of the first measurement point A from the start of measurement and six consecutive measurement points immediately after the first measurement point A (that is, a total of 7 measurement points existing in a section with a measurement time of 24 seconds). The slope of the resulting approximate expression is defined as the initial viscosity increase rate (unit: /s).

An example of Va and Vb is described in Fig. 1A. The RVA viscosity curve of a typical raw starch, as shown by the solid line, results in a breakdown after the viscosity increases in a gelatinization period, resulting in a peak viscosity. In addition, during the cooling period, the viscosity decreased by breakdown increases again due to retrogradation, and has a minimum value. Therefore, the value of Va-Vb increases for a starch with significant breakdown such as raw starch. On the other hand, as indicated by a broken line, when the viscosity increase in the RVA viscosity curve is monotonous and no breakdown occurs, Va and Vb coincide as in Fig. 1A. In this manner, the value of Va-Vb reflects the degree of breakdown. From the viewpoint of viscosity stability after gelatinization, the smaller Va-Vb is the more preferred.

Fig. 1B is an explanatory view of the initial viscosity increase rate. The initial viscosity increase rate based on the above definition represents the increase rate of $V_{Rel}$ with respect to time at the time when the sample starch granules swell and the viscosity starts to increase. Therefore, it is shown that the smaller the initial viscosity increase rate, the more slowly the swelling of the sample starch occurs. When the swelling of the sample starch is moderate, collapse due to rapid swelling of starch granules hardly occurs. Therefore, from the viewpoint of the viscosity stability after gelatinization, it is preferable that the initial viscosity increase rate is small.

The modified starch of the present invention preferably has the following characteristics (V) from the viewpoint of remarkably exhibiting the effects of the present invention. It is easily understood that the modified starch having such characteristics is excellent in high temperature heating and long-term storage.

Characteristics (V): The viscosity of a gelatinized starch paste obtained from a water suspension containing 5 mass% of the modified starch of the present invention is defined as follows.

The physically modified starch in which the relative value A of viscosities before and after the retort sterilization treatment is, for example, 80% or more, preferably 90% or more, and is, for example, 500% or less, 400% or less, 300% or less, 200% or less, or 150% or less, and the relative value B of viscosities during storage after the retort sterilization treatment is, for example, 85% or more, preferably 90% or more, and is, for example, 125% or less, preferably 110% or less,

the physically modified starch:

$$\text{relative value A of viscosities before and after retort sterilization treatment (\%)} = \eta_2/\eta_1 \times 100,$$

$$\text{relative value B of viscosities during storage after retort sterilization treatment (\%)} = \eta_3/\eta_2 \times 100;$$

wherein

the water suspension containing 5 mass% of the physically modified starch is heated in a hot water bath while being stirred, kept at 90°C for 10 minutes after reaching 90°C, and then cooled in a constant temperature water bath at 20°C for 1 hour, and then the viscosity measured at 60 rpm using a B-type rotational viscometer is defined as $\eta_1$,
the water suspension containing 5 mass% of the physically modified starch is heated in a hot water bath while being stirred, kept at 90°C for 10 minutes after reaching 90°C, further subjected to a retort sterilization treatment under the conditions of 121°C for 20 minutes, allowed to cool at 20°C for 1 day, and then the viscosity measured at 60 rpm using a B-type rotational viscometer is defined as $\eta_2$,
the water suspension containing 5 mass% of the physically modified starch is heated in a hot water bath while being stirred, kept at 90°C for 10 minutes after reaching 90°C, further subjected to a retort sterilization treatment under the conditions of 121°C for 20 minutes, allowed to cool at 20°C for 1 day, then stored in a constant temperature chamber at 5°C for 1 week (7 days), and temperature-controlled by being immersed in a constant temperature water bath at 20°C for 1 hour, and then the viscosity measured at 60 rpm using a B-type rotational viscometer is defined as $\eta_3$.

The modified starch containing the plant-derived starch of the present invention has any one characteristic or two or more characteristics of the Characteristics (I) to (V),

among them, it is preferable to contain (V) alone; or at least one of (I) and (II) or (III) and (IV),
it is more preferable to have any combination of (I), (II) and (V); (III), (IV) and (V); or all of (I) to (V), and
it is further preferable to have all of (I) to (V).

In the present specification, the B-type rotational viscometer used for viscosity measurement is preferably BL type manufactured by TOKYO KEIKI INC. As the measurement rotor, an attached rotor is selected according to the viscosity of the sample, and No. 1 rotor is used when the viscosity is less than 100 mPa·s, No. 2 rotor is used when the viscosity is 100 to 500 mPa·s, No. 3 rotor is used when the viscosity is 500 to 2000 mPa·s, and No. 4 rotor is used when the viscosity is 2000 mPa·s or more.

[Formulation containing physically modified starch, food or beverage product]

The formulation or food or beverage product of the present invention contains the physically modified starch.

(Formulation)

In the formulation of the present invention, the content of the physically modified starch is not particularly limited, and may be, for example, 10 mass% or more, 30 mass% or more, or 50 mass% or more.

The formulation of the present invention further contains one kind or two or more kinds of a water-soluble polysaccharide; a water-insoluble polysaccharide; an excipient; an emulsifier food; a nutritional component that can be used in a food or beverage product, a pharmaceutical product, a quasi-pharmaceutical product, or a cosmetic product; salts; a taste component; a fruit juice; a flesh of fruit; vegetables; a vegetable juice; an alcohol; an alcoholic liquor; a puree; an extract; a spice; a sweetener; a sugar alcohol; a high-intensity sweetener; a bittering agent; an acidulant; a flavor; a food coloring, and other food additives; and an active ingredient or additive for a pharmaceutical, a quasi-pharmaceutical product, or a cosmetic product; an antiseptics; an antifungal agent, a surfactant, a gelling agent, a solvent, a fragrance, a bactericide, and a deodorizing component may be contained as a component(s) other than the physically modified starch.

The modified starch contained in the formulation of the present invention may be gelatinized. Such a gelatinized formulation is not particularly limited, but is obtained, for example, by the following steps.

(i) The suspension of the modified starch itself is heated by drum drying, spray drying, extruder, or spray cooking to be gelatinized, and dried as it is to prepare a gelatinized modified starch. Thereafter, a component(s) other than the modified starch is optionally added to the gelatinized modified starch to be formulated.
(ii) A water suspension containing the modified starch and optionally the component other than the modified starch is heated by drum drying, spray drying, extruder, or spray cooking to be gelatinized. The obtained formulation liquid containing the gelatinized modified starch is formulated.

The formulation of the present invention is not particularly limited, and can be used for a food or beverage product, a pharmaceutical product, a quasi-pharmaceutical product, a cosmetic product, and daily necessities, but is preferably used for a food or beverage product, a pharmaceutical product, a quasi-pharmaceutical product, or a cosmetic product, and is more preferably used for a food or beverage product. Preferred forms of the food or beverage product are the same as the specific examples mentioned in the section of food or beverage product described later.

The formulation of the present invention is preferably used for production of various foods and beverages as a quality improver or a texture improver for foods and beverages.

(Food and beverage)

The form of the food or beverage product of the present invention can be usually a food product using starch or a food product containing starch, and is preferably a food product produced by a production method including a step in which starch is stirred in water (or in the presence of free water), a step in which starch is gelatinized in water (or in the presence of free water), and a step in which starch is heated in water. Specific examples of such food or beverage product are not particularly limited, but for example, batter; sauce (e.g., a white sauce, a fruit sauce, a fruit preparation), sauces, sweet vinegar starchy sauce; soup; dressing (e.g., a mayonnaise type dressing); daily products such as yoghurt (e.g., a fat-free yogurt, a low-fat yogurt), cheese, and sour cream; noodles such as Japanese wheat noodles, buckwheat noodles, spaghetti, macaroni, and Chinese noodles (The noodles can be, for example, raw noodles, semi-raw noodles, frozen noodles, dried noodles, fried noodles, or non-fried noodle); breads (e.g., bread, whole grain bread); cake and baked confectionery such as cookie; frozen confectionery such as ice cream, ice milk, and lact ice; flour paste (paste containing flour) and custard-type cream; Japanese confectionery such as rice dumpling, bean paste, and sweet beans jelly (yokan); a thin, flat cake of unsweetened batter fried with various ingredients (okonomiyaki), octopus dumpling (tako-yaki), Korean pancake (chijimi), and burrito; jiao-zi (gyoza), spring roll (harumaki), and Chinese steamed bun; fish meat/processed meat products such as hams and sausages; simmered foods such as nishime, food stewed in soy sauce and sugar (kanro-ni), food boiled in water (yu-ni), braised food (uma-ni), and boiled beans (ni-mame); roasted products such as stir-fries, spit-roasted food (kushi-yaki), grilled food (ami-yaki), food wrapped in a foil and baked (hoiru-yaki), and broiled eel or similar long slender fish (kaba-yaki); fried food such as deep-fried food, deep-fried fish and vegetables in a light batter (tempura), and fried food; steamed foods such as shumai and steamed egg hotchpotch (chawanmushi); dressed foods such as dish dressed with sesame sauce (goma-ae); salads; western confectionery such as pudding, jelly, bavarian cream, mousse, and almond jelly; powder mixes such as pancake mix; instant foods such as instant soup; egg product (e.g., rolled egg, scrambled egg); beverages (e.g., milk beverage, coffee beverage, black tea beverage, fruit juice beverage).

The food or beverage product of the present invention further contains one kind or two or more kinds of a water-soluble polysaccharide; a water-insoluble polysaccharide; an excipient; an emulsifier food; a nutritional component that can be used in a food or beverage product; salts; a taste component; a fruit juice; a flesh of fruit; vegetables; a vegetable juice; an alcohol; an alcoholic liquor; a puree; an extract; a spice; a sweetener; a sugar alcohol; a high-intensity sweetener; a bittering agent; an acidulant; a flavor; a food coloring, and other food additives may be contained as a component(s) other than the physically modified starch.

The food or beverage product of the present invention is preferably heated during the production or use (cooking) of the food or beverage product from the viewpoint of remarkably exhibiting the effects of the present invention. The heating is not particularly limited, and examples thereof include heating at 90°C for 10 minutes or more and heating at 121°C for 4 minutes or more.

EXAMPLES

Hereinafter, the present invention is described in more detail with reference to Examples, but the present invention is not limited thereto. Unless otherwise specified, "part" means "parts by mass", and "%" means "mass%". In addition, the mark "*" in the sentence indicates that it is manufactured by San-Ei Gen F.F.I., Inc. and the mark "※" in the sentence indicates that it is a registered trademark of San-Ei Gen F.F.I., Inc.

Hereinafter, in Test Examples, an example having a number with "R" at the end is a raw starch (Comparative Example), and an example having a number with "NF" is a modified starch containing no a plant-derived dietary fiber or a mushroom-derived dietary fiber (Comparative Example). Examples having numbers with "A" and "B" at the end represent Method A or B of the method for adding a plant-derived dietary fiber or a mushroom-derived dietary fiber described in the following method. Further, the example having the number with "Acid" at the end represents a modified starch in which the pH of the mixture for heat treatment was less than 5 (Comparative Example).

[Common Method]

(Method for preparing modified starch)

In Test Examples described later, a mixture for heat treatment was prepared by adding a plant-derived dietary fiber or a mushroom-derived dietary fiber by the following two methods (Method A and B). The obtained mixture for heat treatment was spread on a tray, dried at 40°C, and pulverized. Then, the resulting pulverized product was passed through a sieve with a mesh size of 150 μm to obtain a passing fraction. The obtained passed fraction was further subjected to dry heat treatment. The type and addition ratio of each component used, the method of adding a plant-derived dietary fiber or a mushroom-derived dietary fiber, and the heat treatment conditions are described in each table. The treated sample was taken out into a stainless steel tray and dried at room temperature overnight. Then, the sample was pulverized in a mortar to obtain a uniform powder.

Method A: (i) 100 g of starch, a plant-derived dietary fiber or a mushroom-derived dietary fiber and optionally a basic compound were mixed with 150 g of water to obtain a suspension. (ii) The suspension was dried to obtain a dried product, and the dried product was used as a mixture for heat treatment.

Method B: (i) 100 g of starch and optionally a basic compound were mixed with 150 g of water to obtain a suspension. (ii) The suspension was dried to obtain a dried product. (iii) A plant-derived dietary fiber powder or a mushroom-derived dietary fiber powder was added to the dried product to obtain a mixture for heat treatment.

In the following Test Examples, the pH of the mixture for heat treatment is a measured value of pH at 25°C when 5 g of each mixture for heat treatment is suspended in 95 ml of water.

(Evaluation of swelling and/or collapse suppressing effects of modified starch using rapid viscoanalyzer (RVA))

For each sample, the swelling and/or collapse suppressing effects was examined using RVA Super 3 manufactured by Newport Scientific for measurement of the following Profile 1 and RVA-4800 manufactured by NSP Perten for measurement of Profile 2. RVA is a device that can continuously measure the viscosity of a test sample while heating and cooling the test sample at a programmed temperature and stirring (rotating speed). In RVA-4800, it is possible to use the HT mode, which measures the sample while the sample is heated to 100°C or higher in a sealed state.

(1) The sample prepared above was subjected to the RVA, and the viscosity was measured over time under the following conditions (Profile 1, maximum temperature 95°C) or (Profile 2, maximum temperature 120°C).

<RVA measurement conditions (Profile 1, maximum temperature 95°C)>

(a) An ion-exchanged water was added to 1.2 g of a sample to make the total amount 25 g, thereby preparing a slurry.
(b) The viscosity of the slurry was measured using RVA under following conditions during the period of 0 to 780 seconds, and

the conditions are that
the sample temperature is held at 50°C for the hydration period of 0 to 60 seconds,
raised at 0.203°C/sec for the gelatinization period of 60 to 282 seconds,
held at 95°C for the high temperature holding period of 282 to 432 seconds,
lowered at 0.197°C/sec for the cooling period of 432 to 660 seconds, and
held at 50°C for the cool holding period of 660 to 780 seconds, and
the rotating speed of the paddle is 960 rpm from 0 to 10 seconds, and 160 rpm after 10 seconds.

<RVA measurement conditions (Profile 2, maximum temperature 120°C)>

(a) An ion-exchanged water was added to 1.2 g of a sample to make the total amount 25 g, thereby preparing a slurry.
(b) The viscosity of the slurry was measured using RVA under following conditions during the period of 0 to 1380 seconds, and

the conditions are that
the sample temperature is held at 50°C for the hydration period of 0 to 60 seconds,
raised at 0.194°C/sec for the gelatinization period of 60 to 420 seconds,
held at 120°C for the high temperature holding period of 420 to 1020 seconds, and
lowered at 0.194°C/sec for the cooling period of 1020 to 1380 seconds, and
the rotating speed of the paddle is 960 rpm from 0 to 10 seconds, and 160 rpm after 10 seconds.

In both conditions of RVA (Profile 1) and RVA (Profile 2), the viscosity between 0 and 60 seconds is not used for evaluation because an artifact occurs due to the start of stirring and measurement. During the measurement period, viscosity data was collected every 4 seconds.

(2) From the obtained RVA viscosity curve, Va-Vb and the initial viscosity increase rate (Vr) were determined according to the following definitions.

(a)Va-Vb

The maximum value of the viscosity during the period from the gelatinization period to the high temperature holding period in the RVA measurement condition is defined as Va, the minimum value of the viscosity during the cooling period is defined as Vb, and the value obtained by subtracting Vb from Va is defined as Va-Vb (unit: mPa·s).

(b)Initial viscosity increase rate (Vr)

In the gelatinization period under the RVA measurement condition, a graph is made in which the vertical axis represents relative viscosity $V_{Rel}$ when Va is 1, and the horizontal axis represents time (seconds). Then, a certain measurement point A on the graph is defined as a measurement point that satisfies the conditions that $V_{Rel}$ increases by 0.01 or more at any of the six consecutive measurement points immediately after A as compared with the measurement point immediately before the point A. At this time, linear approximation is performed on data of the first measurement point A from the start of measurement and six consecutive measurement points immediately after the first measurement point A (that is, a total of 7 measurement points existing in a section with a measurement time of 24 seconds). The slope of the resulting approximate expression is defined as the initial viscosity increase rate (unit: /s).

(Evaluation of residual amount of starch granules in gelatinized starch paste)

(1) A starch sample (gelatinized starch paste) subjected to the measurement of RVA (Profile 1 or Profile 2) was diluted 4 times with an ion-exchanged water to prepare a sample for starch granules observation. A solution of 1% iodine-10% potassium iodide, which is an amount of 1/20 of the sample was added to the sample for starch granules observation to color the starch granule, a digital camera was connected to an optical microscope equipped with an objective lens with a magnification of 10 times, an image was captured in a PC, and the state of the starch granules in a visual field of 1365 μm × 1024 μm was observed.

(2) The residual amount of the starch granules in the gelatinized starch paste was evaluated according to the following evaluation criteria.

<Evaluation criteria of residual amount of starch granule>

0: Starch granules are almost collapsed, and uncollapsed starch granules are present in less than 10% of the visual field.

1: The starch granules are collapsed, and uncollapsed starch granules occupy 10% or more and less than 30% of the visual field.

2: A part of the starch granules are collapsed, and uncollapsed starch granules occupy 30% or more and less than 50% of the visual field.

3: The starch granules are hardly collapsed, and uncollapsed starch granules occupy 50% or more of the visual field.

(Evaluation of long-term stability of viscosity of modified starch)

(1) An ion-exchanged water was added to 10 g of the modified starch sample prepared above to make the total amount 200 g, and a water suspension containing 5 mass% of the modified starch was prepared. The obtained water suspension was heated in a hot water bath to reach 90°C while being stirred, and then kept at 90°C for 10 minutes. Then, the resulting gelatinized starch paste was divided into 100 g portions, and one of the portions was placed in a glass bottle with a lid, cooled in a constant temperature water bath at 20°C for 1 hour, and then stored in a constant temperature chamber at 5°C. The other gelatinized starch paste was filled in a pouch, subjected to a retort sterilization treatment at 121°C for 20 minutes, left at 20°C for 1 day, and then stored at 5°C in a constant temperature chamber.

(2) The viscosity of the sample under the above 2 heating conditions was measured at 20°C and 60 rpm using a B-type rotational viscometer (BL type manufactured by TOKYO KEIKI INC.) before the start of storage at 5°C and 1 week after the start of storage at 5°C. As the measurement rotor, the attached rotor was selected according to the viscosity of the sample. That is, No. 1 rotor is used when the viscosity is less than 100 mPa·s, No. 2 rotor is used when the viscosity is 100 to 500 mPa·s, No. 3 rotor is used when the viscosity is 500 to 2000 mPa·s, and No. 4 rotor is used when the viscosity is 2000 mPa·s or more.

(3) Then, relative values A and B of viscosities with respect to the viscosity of the sample heated at 90°C for 10 minutes before the start of storage at 5°C were determined by the following formula. When the relative value of the viscosity is more than 100%, it indicates that the viscosity has increased from before the start of storage at 5°C, and when the relative value is less than 100%, it indicates that the viscosity has decreased from before the start of storage at 5°C.

<Relative values of viscosities A and B>

$$\text{Relative value A of viscosities before and after retort sterilization treatment (\%)} = \eta_2/\eta_1 \times 100,$$

wherein

$$\text{Relative value B of viscosities during storage after retort sterilization treatment (\%)} = \eta_3/\eta_2 \times 100;$$

(wherein the water suspension containing 5 mass% of the physically modified starch is heated in a hot water bath while being stirred, kept at 90°C for 10 minutes after reaching 90°C, and then cooled in a constant temperature water bath at 20°C for 1 hour, and then the viscosity measured at 60 rpm using a B-type rotational viscometer is defined as $\eta_1$,

the water suspension containing 5 mass% of the physically modified starch is heated in a hot water bath while being stirred, kept at 90°C for 10 minutes after reaching 90°C, further subjected to a retort sterilization treatment under the conditions of 121°C for 20 minutes, allowed to cool at 20°C for 1 day, and then the viscosity measured at 60 rpm using a B-type rotational viscometer is defined as $\eta_2$,

the water suspension containing 5 mass% of the physically modified starch is heated in a hot water bath while being stirred, kept at 90°C for 10 minutes after reaching 90°C, further subjected to a retort sterilization treatment under the conditions of 121°C for 20 minutes, allowed to cool at 20°C for 1 day, then stored in a constant temperature chamber at 5°C for 1 week (7 days), and temperature-controlled by being immersed in a constant temperature water bath at 20°C for 1 hour, and then the viscosity measured at 60 rpm using a B-type rotational viscometer is defined as $\eta_3$.)

(4) When the relative value A of viscosities before and after retort sterilization is 80% or more, it is determined that the property that the viscosity does not easily decrease even when the sample is heated at a high temperature is good. When the relative value B of viscosities during storage is in the range of 85 to 125%, it is determined that the stability of the viscosity after gelatinization is good.

(Fracture test of starch gel)

(1) The water suspension containing 10 mass% of the modified starch sample prepared above was heated to 90°C while being stirred, and then kept at this temperature for 10 minutes to be gelatinized. A part of the resulting gelatinized starch paste was directly filled in a plastic cup (diameter: 35 mm, height: 35 mm) and stored in a constant temperature chamber at 5°C for 1 day. Of the remaining gelatinized starch paste, 100 g was filled into a pouch and then subjected to retort sterilization treatment under the condition of 121°C for 20 minutes. The gelatinized starch paste subjected to retort sterilization treatment was immediately filled in a plastic cup and stored at 5°C for 1 day.

(2) The sample stored at 5°C for 1 day in a refrigerator was taken out and immediately subjected to a fracture test using a texture analyzer (TA-XT2i manufactured by Stable Micro Systems). The cup containing the sample stored at 5°C was placed with the top (opening) facing upward on a horizontal surface stage installed immediately below a plunger having a bottom area of 1 $cm^2$, and the plunger was moved vertically downward at a speed of 1 mm/sec. While the plunger was inserted and moved to have a clearance of 5 mm after contact with the upper surface of the sample, the fracture of the gel was evaluated as (-) when the load value applied to the bottom surface of the plunger monotonically increased, and the fracture of the gel was evaluated as (+) when the load value decreased during the insertion movement.

[Test Example 1: Modification of starch by plant-derived dietary fiber and evaluation of characteristics thereof]

Various raw material starches (raw starches) were treated under the conditions shown in Table 1 below. The obtained modified starches were subjected to RVA measurement under the conditions of RVA (Profile 1, maximum temperature 95°C), and Va-Vb, the initial viscosity increase rate, and the residual amount of starch granules were evaluated based on the above methods. The results are shown in the same Table 1. The RVA viscosity curves of Comparative Example 1-1-R and NF and Examples 1-1-A and B are shown in Fig. 2. Some modified starches or raw material starches thereof were subjected to RVA measurement under the conditions of Profile 2 (maximum temperature 120°C), and Va-Vb, the initial viscosity increase rate, and the residual amount of starch granules were evaluated based on the above methods. The results are shown in Table 2. The RVA viscosity curve is shown in Fig. 3.

Even when any raw starch was used as a raw material, the samples of Examples obtained by adding a plant-derived dietary fiber and performing the modification treatment by Method A or B had a Va-Vb of 100 mPa·s or less, which was lower than that of Comparative Examples in which the dry heat treatment was performed without adding a plant-derived dietary fiber, and suppressed the breakdown. In addition, the modified starch of Examples had an initial viscosity increase rate Vr of 0.02/s or less, which was lower than that of Comparative Examples.

As shown in Figs. 2 and 3, in the waxy corn starch, a clear viscosity peak appeared by heating and a minimum value was shown in the cooling process in the raw waxy corn starch before modification (Comparative Example 1-1-R) and Comparative Example 1-1-NF, so that clear breakdown was observed. On the other hand, the modified waxy corn starches of Examples 1-1-A and B and Examples 1-7-A and 1-8-A prepared by dry heat treatment in a state of containing both a dietary fiber and a basic compound had no breakdown.

The results of observing the starch granules of some starch samples (gelatinized starch pastes) after subjected to the measurement of RVA (Profile 1 or Profile 2) are shown in Table 1, Table 2, and Fig. 4. As shown in Fig. 4, in Comparative Example 1-1-R and Comparative Example 1-1-NF, collapse of starch granules was often observed after heating at a maximum of 95°C in RVA Profile 1, and uncollapsed starch granules were observed in only less than 30% of the visual field. On the other hand, in Examples 1-1-A, 1-7-A, and 1-8-A, even after heating at a maximum of 120°C in RVA Profile 2, many starch granules having a large particle size remained, and 30 to 50% or more of the visual field was occupied by uncollapsed starch granules.

[Table 1]

| Sample name | Production conditions | | | | | | | | | Evaluation | | | | |
|---|---|---|---|---|---|---|---|---|---|---|---|---|---|---|
| | (A) Starch | (B) Plant-derived dietary fiber | | (C) Basic compound | | Methods of adding (B) | pH of mixture for heat treatment | Dry heat temperature (°C) | Dry heating time (min) | Va (mPa·s) | Vb (mPa·s) | Va-Vb (mPa·s) | Initial viscosity increase rate $Vr_1$ (/s) | Residual amount of starch granules |
| | | Type | Mass% | Type | Mass% | | | | | | | | | |
| Comparative Example 1-1-R | Waxy corn starch | - | - | - | - | - | 4.40 | No heat treatment | | 671 | 436 | 235 | 0.0166 | 0 |
| Comparative Example 1-1-NF | | - | - | $Na_2CO_3$ | 0.0225 | - | 5.76 | 180 | 60 | 358 | 251 | 107 | 0.0222 | 1 |
| Example 1-1-A | | CF | 5 | $Na_2CO_3$ | 0.1065 | Method A | 6.26 | 180 | 60 | 218 | 217 | 1 | 0.0111 | 3 |
| Example 1-1-B | | CF | 5 | $Na_2CO_3$ | 0.1065 | Method B | 6.19 | 180 | 60 | 579 | 578 | 1 | 0.0132 | 3 |
| Comparative Example 1-2-R | Tapioca starch | - | - | - | - | - | 5.03 | No heat treatment | | 459 | 375 | 84 | 0.0213 | 0 |
| Example 1-2-A | | CF | 5 | $Na_2CO_3$ | 0.0965 | Method A | 6.46 | 160 | 60 | 326 | 293 | 33 | 0.0056 | 2 |
| Example 1-2-B | | CF | 5 | $Na_2CO_3$ | 0.0965 | Method B | 5.80 | 160 | 60 | 352 | 347 | 5 | 0.0075 | 2 |
| Comparative Example 1-3-R | Potato starch | - | - | - | - | - | 7.13 | No heat treatment | | 3109 | 1317 | 1792 | 0.0297 | 0 |
| Comparative Example 1-3-NF | | - | - | - | - | - | 7.15 | 160 | 60 | 802 | 433 | 369 | 0.0171 | 1 |
| Example 1-3-A | | CF | 5 | $Na_2CO_3$ | 0.069 | Method A | 6.58 | 160 | 60 | 698 | 697 | 1 | 0.0030 | 3 |
| Example 1-3-B | | CF | 5 | $Na_2CO_3$ | 0.069 | Method B | 6.69 | 160 | 60 | 876 | 876 | 0 | 0.0031 | 2 |
| Example 1-4-A | Waxy rice starch | CF | 5 | $Na_2CO_3$ | 0.0805 | Method A | 6.51 | 160 | 60 | 304 | 304 | 0 | 0.0115 | 3 |
| Example 1-4-B | | CF | 5 | $Na_2CO_3$ | 0.0805 | Method B | 6.16 | 160 | 60 | 302 | 300 | 2 | 0.0157 | 3 |

(continued)

| Sample name | Production conditions | | | | | | | | | Evaluation | | | | |
|---|---|---|---|---|---|---|---|---|---|---|---|---|---|---|
| | (A) Starch | (B) Plant-derived dietary fiber | | (C) Basic compound | | Methods of adding (B) | pH of mixture for heat treatment | Dry heat temperature (°C) | Dry heating time (min) | Va (mPa·s) | Vb (mPa·s) | Va-Vb (mPa·s) | Initial viscosity increase rate $Vr_1$ (/s) | Residual amount of starch granules |
| | | Type | Mass% | Type | Mass% | | | | | | | | | |
| Comparative Example 1-5-NF | Waxy corn starch | - | - | $Na_2CO_3$ | 0.0225 | - | 5.76 | 160 | 60 | 575 | 466 | 109 | 0.0196 | 1 |
| Example 1-5-A | | CF | 5 | $Na_2CO_3$ | 0.1065 | Method A | 6.26 | 160 | 60 | 276 | 276 | 0 | 0.0135 | 3 |
| Example 1-5-B | | CF | 5 | $Na_2CO_3$ | 0.1065 | Method B | 6.19 | 160 | 60 | 482 | 482 | 0 | 0.0123 | 3 |
| Comparative Example 1-6-NF | Potato starch | - | - | - | - | - | 7.15 | 160 | 20 | 1766 | 1377 | 389 | 0.0207 | 1 |
| Example 1-6-A | | CF | 5 | $Na_2CO_3$ | 0.069 | Method A | 6.58 | 160 | 20 | 1114 | 1082 | 32 | 0.0056 | 3 |
| Example 1-6-B | | CF | 5 | $Na_2CO_3$ | 0.069 | Method B | 6.69 | 160 | 20 | 1036 | 1036 | 0 | 0.0069 | 3 |
| Example 1-7-A | Waxy corn starch | SF | 1 | $Na_2CO_3$ | 0.029 | Method A | 6.28 | 160 | 60 | 339 | 338 | 1 | 0.0107 | 2 |
| Example 1-8-A | Waxy corn starch | CF | 5 | $Na_2CO_3$ | 0.1065 | Method A | 6.26 | 160 | 180 | 102 | 102 | 0 | 0.0073 | 3 |

1) CF: Citrus fiber, SF: soybean-derived dietary fiber
2) A kneader was used for heating at 180°C. A constant temperature dry heat device is used for heating at 160°C.
3) The mass% represents the amount added with respect to the amount of starch.

[Table 2]

| Sample name | Production conditions | | | | | | | | | Evaluation | | | | |
|---|---|---|---|---|---|---|---|---|---|---|---|---|---|---|
| | (A) Starch | (B) Plant-derived dietary fiber | | (C) Basic compound | | Methods of adding (B) | pH of mixture for heat treatment | Dry heat temperature (°C) | Dry heating time (min) | Va (mPa·s) | Vb (mPa·s) | Va-Vb (mPa·s) | Initial viscosity increase rate Vr (/s) | Residual amount of starch granules |
| | | Type | Mass% | Type | Mass% | | | | | | | | | |
| Comparative Example 1-1-R | Waxy corn starch | - | - | - | - | - | 4.40 | No heat treatment | | 466 | 118 | 348 | 0.0186 | 0 |
| Comparative Example 1-1-NF | | - | - | $Na_2CO_3$ | 0.0225 | - | 5.76 | 180 | 60 | 283 | 90 | 193 | 0.0199 | 1 |
| Example 1-1-A | | CF | 5 | $Na_2CO_3$ | 0.1065 | Method A | 6.26 | 180 | 60 | 197 | 166 | 31 | 0.0074 | 2 |
| Example 1-2-B | Tapioca starch | CF | 5 | $Na_2CO_3$ | 0.0965 | Method B | 5.80 | 160 | 60 | 219 | 120 | 99 | 0.0106 | 2 |
| Comparative Example 1-3-NF | Potato starch | - | - | - | - | - | 7.15 | 160 | 60 | 501 | 49 | 452 | 0.0180 | 0 |
| Example 1-4-B | Waxy rice starch | CF | 5 | $Na_2CO_3$ | 0.0805 | Method B | 6.16 | 160 | 60 | 252 | 235 | 17 | 0.0151 | 3 |
| Example 1-5-B | Waxy corn starch | CF | 5 | $Na_2CO_3$ | 0.1065 | Method B | 6.19 | 160 | 60 | 340 | 304 | 36 | 0.0130 | 3 |
| Example 1-7-A | Waxy corn starch | SF | 1 | $Na_2CO_3$ | 0.029 | Method A | 6.28 | 160 | 60 | 235 | 218 | 17 | 0.0157 | 2 |
| Example 1-8-A | Waxy corn starch | CF | 5 | $Na_2CO_3$ | 0.1065 | Method A | 6.26 | 160 | 180 | 175 | 173 | 2 | 0.0067 | 2 |

1) CF: Citrus fiber, SF: soybean-derived dietary fiber
2) A kneader was used for heating at 180°C. A constant temperature dry heat device is used for heating at 160°C.
3) The mass% represents the amount added with respect to the amount of starch.

From the above, it became clear that the modified starch of Examples suppressed the collapse of starch granules during gelatinization by heating. It is presumed that the modified starch of the present invention has starch granules that are resistant to such heating, and thus has a property that viscosity is exhibited while the viscosity is hardly decreased over time.

From the results in Table 1, it is inferred that when the initial viscosity increase rate is low, gelatinization occurs slowly, so that collapse due to rapid swelling of starch granules is suppressed, and as a result, many uncollapsed starch granules are observed with a microscope.

[Test Example 2. Evaluation of storage stability of modified starch]

(1) As shown in Table 3, a part of the modified starch described in Table 1 above and Comparative Example 1-Acid were subjected to long-term stability evaluation of the viscosity.

As a result, when the pH of the mixture for heat treatment to which the plant-derived dietary fiber was added was 5 or more, the viscosity ($\eta_1$) by heating at 90°C for 10 minutes was 200 mPa·s or more. Moreover, when the sample heated and dissolved at 90°C was further subjected to a high temperature retort sterilization treatment, the viscosity was maintained or increased as compared with the viscosity by heating at 90°C, and the relative value A of viscosities before and after retort sterilization was 80% or more. The viscosity was stable even after the retort sterilization treatment and storage at 5°C for 1 week, and the relative value B of viscosities during storage after the retort sterilization was within the range of 85 to 125%. On the other hand, when a plant-derived dietary fiber was not contained or when dry heat treatment was performed at less than pH 5, significant viscosity decrease was observed by retort sterilization treatment.

From the above, it was shown that the modified starch of Examples did not decrease in viscosity even under the retort sterilization treatment conditions, and the viscosity after gelatinization hardly decreased even after long-term storage for 1 week. In addition, it has been revealed that the modified starch prepared by Method A has a small increase in viscosity after being subjected to retort sterilization treatment and then stored at 5°C for 1 week, and has excellent temporal stability of viscosity, as compared with the modified starch prepared by Method B.

[Table 3]

| Sample name | (A) Starch | (B) Plant-derived dietary fiber | (C) Basic compound | Methods of adding (B) | pH of mixture for heat treatment | Viscosity (mPa·s) | | | | Relative value A of viscosities before and after retort sterilization (%) | Relative value B of viscosities during Storage (%) |
|---|---|---|---|---|---|---|---|---|---|---|---|
| | | | | | | Treatment 90°C for at 10 minutes | | 90°C for 10 minutes + retort sterilization | | | |
| | | | | | | Before storage at 5°C $\eta_1$ | After one week | Before storage at 5°C $\eta_2$ | After one week $\eta_3$ | | |
| Comparative Example 1-1-R | Waxy corn starch | - | - | - | 4.40 | 920 | 1351 | 305.3 | 394.5 | 33 | 129 |
| Comparative Example 1-1-NF | | - | $Na_2CO_3$ | - | 5.76 | 591 | 498.9 | 293.8 | 340.1 | 50 | 116 |
| Example 1-5-A | | CF | $Na_2CO_3$ | Method A | 6.26 | 1186 | 1278 | 1249 | 1249 | 105 | 100 |
| Example 1-5-B | | CF | $Na_2CO_3$ | Method B | 6.19 | 2220 | 2740 | 2700 | 2780 | 122 | 103 |
| Comparative Example 1-4-NF | Waxy rice starch | - | - | - | 6.08 | 174.6 | 210.6 | 131.2 | 136.1 | 75 | 104 |
| Example 1-4-A | | CF | $Na_2CO_3$ | Method A | 6.51 | 1594 | 1740 | 1760 | 1910 | 110 | 109 |
| Example 1-4-B | | CF | $Na_2CO_3$ | Method B | 6.16 | 1749 | 1792 | 1760 | 2160 | 101 | 123 |
| Example 1-7-A | Waxy corn starch | SF | $Na_2CO_3$ | Method A | 6.28 | 1585 | 1743 | 1689 | 1690 | 107 | 100 |

(continued)

| Sample name | (A) Starch | (B) Plant-derived dietary fiber | (C) Basic compound | Methods of adding (B) | pH of mixture for heat treatment | Viscosity (mPa·s) | | | | Relative value A of viscosities before and after retort sterilization (%) | Relative value B of viscosities during Storage (%) |
|---|---|---|---|---|---|---|---|---|---|---|---|
| | | | | | | Treatment 90°C for at 10 minutes | | 90°C for 10 minutes + retort sterilization | | | |
| | | | | | | Before storage at 5°C $\eta_1$ | After one week | Before storage at 5°C $\eta_2$ | After one week $\eta_3$ | | |
| Comparative Example 1-Acid | Waxy corn starch | CF | - | Method A | 3.90 | 140.8 | 141.9 | 31.7 | 32.5 | 23 | 103 |

1) CF: Citrus fiber SF: soybean-derived dietary fiber
2) The mass% represents the amount added with respect to the amount of starch.
3) Relative value A of viscosities before and after retort sterilization (%) = $\eta_2/\eta_1 \times 100$, Relative value B of viscosities during storage (%) = $\eta_3/\eta_2 \times 100$

(2) A potato starch and a tapioca starch may form a gel. Therefore, starch gels derived from these starches among the modified starch samples in Table 1 were prepared by the method, and the presence or absence of fracture and the texture were evaluated. As an evaluation of the presence or absence of fracture, an evaluation using the texture analyzer was performed. In addition, the characteristics of the gel were evaluated by a sensory evaluation by eating.

The results are shown in Table 4. Both the potato starch and the tapioca starch subjected to dry heat treatment in the presence of a plant-derived dietary fiber formed gel without fracture and with soft texture. It is presumed that such a change in physical properties is caused by suppression of retrogradation during low-temperature storage by the modification by the method of the present invention.

[Table 4]

| Sample name | Starch | Retort sterilization | Fracture | Gel characteristics |
|---|---|---|---|---|
| Comparative Example 1-2-NF | Tapioca starch | No | + | A hard and brittle gel was formed, an unfavorable texture |
| | | With | + | |
| Example 1-2-A | | No | - | An elastic gel was formed |
| | | With | - | |
| Comparative Example 1-3-R | Potato starch | No | + | A hard and brittle gel was formed, an unfavorable texture |
| | | With | + | |
| Comparative Example 1-3-NF | | No | + | A hard and brittle gel was formed, an unfavorable texture |
| | | With | + | |
| Example 1-3-A | | No | - | A soft gel was formed |
| | | With | - | |
| Example 1-3-B | | No | - | A soft gel was formed |
| | | With | - | |

[Test Example 3. Study on type of plant-derived dietary fiber or mushroom-derived dietary fiber in modification treatment and heating conditions]

Raw material starches (raw starches) were treated under the conditions shown in Table 5-1 below. The obtained modified starch was subjected to RVA measurement under the conditions of RVA (Profile 1, maximum temperature 95°C), and Va-Vb, the initial viscosity increase rate Vr, and the residual amount of starch granules were evaluated based on the above methods.

The results are shown in Table 5-2. In all of the modified starches of Examples 2-1 to 2-12, the values of Va-Vb, Vr and the residual ratio of starch granules were good. Further, the modified starches in these examples also have good values of relative value A of viscosities and relative value B of viscosities, and it became clear that the modified starches do not easily lose viscosity even when heated at high temperatures, and have excellent stability after gelatinization. On the other hand, in the case of performing the dry heat treatment without adding the plant-derived dietary fiber or the mushroom-derived dietary fiber (Comparative Example 2-1), both the relative values A and B of viscosities were poor.

As in Example 2-5, the modified starch obtained by the long-time (240 minutes) dry heat treatment at 160°C showed a property that the viscosity significantly increased when further subjected to retort sterilization treatment after treatment at 90°C for 10 minutes. In addition, it has been found that when the dry heating time is longer as in Example 2-5 or Example 2-9, viscosity decrease associated with high temperature heating is further suppressed, and the stability of viscosity after gelatinization tends to be improved.

It was found that when the mixture for heat treatment is prepared to have a pH of 13, the mixture is thickened during mixing, the composition becomes nonuniform, and a modified starch of sufficient quality cannot be obtained.

[Table 5-1]

| Sample name | Production conditions | | | | | | | | |
|---|---|---|---|---|---|---|---|---|---|
| | (A) Starch | (B) Plant-derived dietary fiber or mushroom-derived dietary fiber | | (C) Basic compound | | Methods of adding (B) | pH of mixture for heat treatment | Dry heat temperature (°C) | Dry heating time (min) |
| | | Type | Mass% | Type | Mass% | | | | |
| Comparative Example 2-1 | Waxy corn starch | No | 0 | NaOH | 0.0313 | Method B | 7.29 | 160 | 60 |
| Example 2-1 | Waxy corn starch | Sugar beet-derived dietary fiber | 5 | $Na_2CO_3$ | 0.1065 | Method A | 5.01 | 180 | 60 |
| Example 2-2 | Waxy corn starch | Pea-derived dietary fiber | 5 | NaOH | 0.0313 | Method B | 6.96 | 160 | 60 |
| Example 2-3 | Waxy corn starch | Bamboo-derived dietary fiber | 5 | NaOH | 0.0313 | Method B | 7.00 | 160 | 60 |
| Example 2-4 | Waxy corn starch | Apple-derived dietary fiber | 5 | $Na_2CO_3$ | 0.5194 | Method B | 9.01 | 160 | 60 |
| Example 2-5 | Waxy corn starch | CF | 5 | $Na_2CO_3$ | 0.1723 | Method B | 6.98 | 160 | 240 |
| Example 2-6 | Waxy corn starch | CF | 10 | $Na_2CO_3$ | 0.5194 | Method B | 7.35 | 160 | 60 |
| Example 2-7 | Waxy corn starch | CF | 5 | $Na_2CO_3$ | 0.5194 | Method B | 9.33 | 160 | 30 |
| Example 2-8 | Waxy corn starch | CF | 5 | $Na_2CO_3$ | 0.6480 | Method B | 10.34 | 160 | 30 |

(continued)

| Sample name | Production conditions | | | | | | | | |
|---|---|---|---|---|---|---|---|---|---|
| | (A) Starch | (B) Plant-derived dietary fiber or mushroom-derived dietary fiber | | (C) Basic compound | | Methods of adding (B) | pH of mixture for heat treatment | Dry heat temperature (°C) | Dry heating time (min) |
| | | Type | Mass% | Type | Mass% | | | | |
| Example 2-9 | Waxy corn starch | CF | 5 | $Na_2CO_3$ | 0.5194 | Method B | 9.29 | 120 | 540 |
| Example 2-10 | Waxy corn starch | White jelly fungus-derived dietary fiber | 1 | $Na_2CO_3$ | 0.025 | Method A | 5.30 | 160 | 120 |
| Example 2-11 | Waxy corn starch | Basil seed-derived dietary fiber | 0.5 | $Na_2CO_3$ | 0.0265 | Method A | 5.82 | 160 | 60 |
| Example 2-12 | Waxy corn starch | Chia seed-derived dietary fiber | 0.5 | $Na_2CO_3$ | 0.0315 | Method A | 6.02 | 160 | 60 |

1) CF: Citrus fiber
2) A kneader was used for heating at 180°C. A constant temperature dry heat device is used for heating at 160°C.
3) The mass%- represents the amount added with respect to the amount of starch.
4) Relative value A of viscosities before and after retort sterilization (%) = $\eta_2/\eta_1 \times 100$, relative value B of viscosities during storage (%) = $\eta_3/\eta_2 \times 100$

[Table 5-2]

| Sample name | Evaluation | | | | | Viscosity (mPa·s) | | | | Relative value A of viscosities before and after retort sterilization (%) | Relative value B of visco sities during Storage (%) |
|---|---|---|---|---|---|---|---|---|---|---|---|
| | Va (mPa·s) | Vb (mPa·s) | Va-Vb (mPa·s) | Initial viscosity rate increase Vr (/s) | Residual amount of starch granules | Treatment at 90°C for 10 minutes | | 90°C for 10 minutes + retort sterilization | | | |
| | | | | | | Before storage at 5°C $\eta_1$ | After one week | Before storage at 5°C $\eta_2$ | After one week $\eta_3$ | | |
| Comparative Example 2-1 | 769 | 621 | 148 | 0.0212 | 1 | 1335 | 1518 | 931 | 1511 | 70 | 162 |
| Example 2-1 | 355 | 344 | 11 | 0.0180 | 3 | 1209 | 1398 | 1238 | 1332 | 102 | 108 |
| Example 2-2 | 569 | 531 | 38 | 0.0148 | 2 | 1294 | 1350 | 1365 | 1327 | 105 | 97 |
| Example 2-3 | 557 | 519 | 38 | 0.0199 | 2 | 1461 | 1546 | 1474 | 1616 | 101 | 110 |
| Example 2-4 | 404 | 403 | 1 | 0.0153 | 3 | 2210 | 2330 | 2450 | 2590 | 111 | 106 |
| Example 2-5 | 85 | 85 | 0 | 0.0089 | 3 | 717 | 834 | 1551 | 1467 | 216 | 95 |
| Example 2-6 | 261 | 261 | 0 | 0.0169 | 3 | 1746 | 1660 | 1882 | 2060 | 108 | 109 |
| Example 2-7 | 674 | 651 | 23 | 0.0189 | 2 | 1301 | 1314 | 1257 | 1405 | 97 | 112 |
| Example 2-8 | 571 | 571 | 0 | 0.0182 | 3 | 1382 | 1676 | 1466 | 1667 | 106 | 114 |
| Example 2-9 | 611 | 599 | 12 | 0.0146 | 2 | 878 | 886 | 998 | 1171 | 114 | 117 |
| Example 2-10 | 319 | 317 | 2 | 0.0145 | 3 | 1434 | 1483 | 1211 | 1211 | 84 | 100 |
| Example 2-11 | 365 | 341 | 24 | 0.0171 | 3 | 1075 | 1026 | 1018 | 1062 | 95 | 104 |
| Example 2-12 | 256 | 256 | 0 | 0.0163 | 3 | 1182 | 1189 | 1279 | 1250 | 108 | 98 |

Further, some of the examples in Table 5-1 above were subjected to RVA measurement even under the conditions of RVA (Profile 2, maximum temperature 120°C), and Va-Vb, the initial viscosity increase rate Vr, and the residual amount of starch granules were evaluated based on the above methods.

The results are shown in Table 5-3. In all of the modified starches of Examples, the values of Va-Vb, Vr and the residual ratio of starch granules were good.

[Table 5-3]

| Sample | Production conditions | | | | | | | | | Evaluation | | | | |
|---|---|---|---|---|---|---|---|---|---|---|---|---|---|---|
| | (A) Starch | (B) Plant-derived dietary fiber or mushroom-derived dietary fiber | | (C) Basic compound | | Methods of adding (B) | pH of mixture for heat treatment | Dry heat temperature (°C) | Dry heating time (min) | Va (mPa·s) | Vb (mPa·s) | Va-Vb (mPa·s) | Initial viscosity increase rate Vr (/s) | Residual amount of starch granules |
| | | Type | Mass% | Type | Mass% | | | | | | | | | |
| Example 2-1 | Waxy corn starch | Sugar beet-derived dietary fiber | 5 | $Na_2CO_3$ | 0.1065 | Method A | 5.01 | 180 | 60 | 203 | 93 | 110 | 0.0180 | 2 |
| Example 2-2 | Waxy corn starch | Pea-derived dietary fiber | 5 | NaOH | 0.0313 | Method B | 6.96 | 160 | 60 | 387 | 250 | 137 | 0.0110 | 2 |
| Example 2-3 | Waxy corn starch | Bamboo-derived dietary fiber | 5 | NaOH | 0.0313 | Method B | 7.00 | 160 | 60 | 383 | 238 | 145 | 0.0195 | 2 |
| Example 2-4 | Waxy corn starch | Apple-derived dietary fiber | 5 | $Na_2CO_3$ | 0.5194 | Method B | 9.01 | 160 | 60 | 300 | 296 | 4 | 0.0189 | 3 |
| Example 2-6 | Waxy corn starch | CF | 10 | $Na_2CO_3$ | 0.5194 | Method B | 7.35 | 160 | 60 | 255 | 250 | 5 | 0.0111 | 3 |
| Example 2-10 | Waxy corn starch | White jelly fungus-derived dietary fiber | 1 | $Na_2CO_3$ | 0.025 | Method A | 5.30 | 160 | 120 | 302 | 170 | 132 | 0.0164 | 3 |

(continued)

| Sample | Production conditions | | | | | | | | | Evaluation | | | | |
|---|---|---|---|---|---|---|---|---|---|---|---|---|---|---|
| | (A) Starch | (B) Plant-derived dietary fiber or mushroom-derived dietary fiber | | (C) Basic compound | | Methods of adding (B) | pH of mixture for heat treatment | Dry heat temperature (°C) | Dry heating time (min) | Va (mPa·s) | Vb (mPa·s) | Va-Vb (mPa·s) | Initial viscosity increase rate Vr (/s) | Residual amount of starch granules |
| | | Type | Mass% | Type | Mass% | | | | | | | | | |
| Example 2-11 | Waxy corn starch | Basil seed-derived dietary fiber | 0.5 | $Na_2CO_3$ | 0.0265 | Method A | 5.82 | 160 | 60 | 321 | 189 | 132 | 0.0149 | 3 |
| Example 2-12 | Waxy corn starch | Chia seed-derived dietary fiber | 0.5 | $Na_2CO_3$ | 0.0315 | Method A | 6.02 | 160 | 60 | 256 | 196 | 60 | 0.0136 | 3 |

1) CF: Citrus fiber
2) A kneader was used for heating at 180°C. A constant temperature dry heat device is used for heating at 160°C.
3) The mass% represents the amount added with respect to the amount of starch.

[Test Example 4. Measurement of swelling degree of various modified starches]

Raw material starches (raw starches) were treated under the conditions shown in Table 6. The swelling degree of the obtained starch sample was measured by the following procedure.

<Measurement procedure of swelling degree>

(1) A starch sample (0.01 g) was dispersed in 5 g of water, and 30 μl of a solution of 1% iodine-10% potassium iodide was added and mixed.
(2) Starch granules were observed under heating with an optical microscope equipped with a hot stage (IMC-0203 manufactured by Imoto machinery Co., LTD.). At 0.1°C/s, the temperature was raised from 25°C to 90°C, 10 or more starch granules having different sizes in an unswollen state at 25°C were selected, and photographed when the temperature reached 25°C and 85°C.
(3) The area occupied by each starch granules in the visual field was measured respectively using Winroof2015 (manufactured by MITANI CORPORATION). As for collapsed starch granules, the region where the collapsed starch was distributed was defined as the area of the starch granules.
(4) When the average values of the areas of the starch granules at 25°C and 85°C (unit: $\mu m^2$) are denoted by $S_{25}$ and $S_{85}$, respectively, the swelling degree (%) of the starch granules is determined by the following formula.

$$\text{Swelling degree (\%)} = (S_{85}/S_{25}) \times 100$$

The results are shown in Table 6. In the modified starch of the present invention of Examples, the swelling degree was decreased to less than 1000%, and it was confirmed that the collapse of starch granules by heating was suppressed as compared with the starch sample of Comparative Example.

[Table 6]

| | (A) Starch | (B) Plant-derived dietary fiber or other water-soluble polysaccharide | Modification conditions | Evaluation of starch granules | | |
|---|---|---|---|---|---|---|
| | | | | Area per granule ($\mu m^2$) | | Swelling degree (%) |
| | | | | 25°C | 85°C | |
| Comparative Example 1-1-R | Waxy corn starch | No | (No modification treatment) | 178 | 2700 | 1518 |
| Comparative Example 1-1-NF | | No | 180°C 60 min | 226 | 3010 | 1332 |
| Comparative Example 1-5-NF | | No | 160°C 60 min | 188 | 2342 | 1243 |
| Comparative Example 3-1 | | No | 160°C 180 min | 239 | 3412 | 1426 |
| Comparative Example 3-2 | | Xanthan gum | 160°C 60 min | 226 | 4378 | 1938 |
| Example 1-5-A | | CF | 160°C 60 min | 206 | 1629 | 791 |
| Example 1-5-B | | CF | 160°C 60 min | 178 | 1668 | 934 |
| Example 1-7-A | | SF | 160°C 60 min | 352 | 3038 | 862 |
| Example 1-8-A | | CF | 160°C 180 min | 274 | 2716 | 991 |
| Comparative Example 1-2-NF | Tapioca starch | No | 160°C 60 min | 201 | 3413 | 1698 |
| Example 1-2-B | | CF | 160°C 60 min | 209 | 1255 | 602 |

(continued)

| | (A) Starch | (B) Plant-derived dietary fiber or other water-soluble polysaccharide | Modification conditions | Evaluation of starch granules | | |
|---|---|---|---|---|---|---|
| | | | | Area per granule (μm$^2$) | | Swelling degree (%) |
| | | | | 25°C | 85°C | |
| Comparative Example 1-3-R | Potato starch | No | (No modification treatment) | 1073 | 12837 | 1196 |
| Comparative Example 1-3-NF | | No | 160°C 60 min | 1212 | 12323 | 1017 |
| Example 1-3-A | | CF | 160°C 60 min | 1410 | 12698 | 900 |
| 1) CF: Citrus fiber, SF: soybean-derived dietary fiber<br>2) All of CF was added in an amount of 5 parts by mass, and all of SF was added in an amount of 1 part by mass with respect to 100 parts by mass of the starch.<br>3) Comparative Example 3-2 was prepared by adding 0.5 parts by mass of a xanthan gum to 100 parts by mass of starch, mixing the mixture, adjusting the moisture so that the moisture content is 17%, and subjecting the mixture to a dry heat treatment. | | | | | | |

[Test Example 5. Shear resistance test of starch sample]

For the gelatinized starch pastes of the starch samples in Table 7, the viscosity change rate when shear was applied by stirring was determined according to the following procedure, and shear resistance was compared.

<Measurement procedure of viscosity decrease rate at the time of shear application>

(1) A starch sample (5 g) was added to 95 g of an ion-exchanged water to prepare a water suspension, and the water suspension was heated in a hot water bath while being stirred at 500 rpm or 1000 rpm. After the temperature reached 90°C, the water suspension was kept at 90°C for 10 minutes. For stirring, a stirrer Model MAZELA NZ (manufactured by TOKYO RIKAKIKAI CO, LTD.) was used.
(2) The resulting gelatinized starch paste was transferred to a glass bottle, cooled in a constant temperature water bath at 20°C for 1 hour, and then the viscosity was measured at 20°C and 60 rpm using a B-type rotational viscometer (BL type manufactured by TOKYO KEIKI INC.). The selection criteria of the rotor are the same as in the "Evaluation of long-term stability of viscosity of modified starch".
(3) The viscosity of the gelatinized starch paste obtained by stirring at 500 rpm is defined as $\eta_4$, and the viscosity of the gelatinized starch paste obtained by stirring at 1000 rpm is defined as $\eta_5$. At this time, $\eta_5/\eta_4$ (%) is obtained by the following formula.

$$\eta_5/\eta_4\ (\%) = \eta_5/\eta_4 \times 100$$

The results are shown in Table 7. In each of the starch samples of Examples, $\eta_5/\eta_4$ was 75 to 150%, and the viscosity did not significantly decrease or slightly increased due to an increase in shear. On the other hand, in the starch sample of Comparative Example, $\eta_5/\eta_4$ significantly decreased, and the viscosity remarkably decreased due to the increase in the shear. Thus, it was found that the physically modified starch of the present invention derived from at least a waxy corn starch, a tapioca starch or a potato starch has excellent shear resistance.

[Table 7]

| Starch sample | (A) Starch | (B) Plant-derived dietary fiber | Modification conditions | Viscosity (mPa·s) | | $\eta_5/\eta_4$ (%) |
|---|---|---|---|---|---|---|
| | | | | After stirring at 500 rpm ($\eta_4$) | After stirring at 1000 rpm ($\eta_5$) | |
| Comparative Example 1-1-NF | Waxy corn starch | No | 180°C 60 min | 712 | 264 | 37 |
| Example 1-1-A | Waxy corn starch | CF | 180°C 60 min | 866 | 888 | 103 |
| Example 1-7-A | Waxy corn starch | SF | 160°C 60 min | 1430 | 1474 | 103 |
| Example 2-2 | Waxy corn starch | Pea-derived dietary fiber | 160°C 60 min | 1700 | 1296 | 76 |
| Example 1-2-A | Tapioca starch | CF | 160°C 60 min | 1150 | 1512 | 131 |
| Example 1-3-A | Potato starch | CF | 160°C 60 min | 1385 | 1310 | 95 |
| 1) CF: Citrus fiber, SF: soybean-derived dietary fiber<br>2) Based on 100 parts by mass of starch, 5 parts by mass of CF and pea-derived dietary fiber, and 1 part by mass of SF are used. | | | | | | |

[Test Example 6. Comparison of viscosity stability of modified starch-containing food]

Chinese starchy sauce of the formulation of Table 8 was prepared. All the raw materials described in the formulation were added, heated for 10 minutes after the temperature reached 90°C, and hot water was added to correct the total amount. The obtained Chinese starchy sauce was filled in a pouch, and retort sterilization was performed at 121°C for 20 minutes. After cooling with flowing water, measurement was performed at 20°C and a rotating speed of 60 rpm using a B-type rotational viscometer. The selection criteria of the rotor are the same as in the "Evaluation of long-term stability of viscosity of modified starch".

The results of the viscosity measurement are shown in Table 8. In Examples containing the modified starch of the present invention, a sufficient thickness suitable for Chinese starchy sauce was obtained, and the flavor was good. On the other hand, the Chinese starchy sauce of the comparative example was decreased in viscosity after being subjected to retort sterilization, and a sufficient thickness was not obtained. From the above, it has become clear that the modified starch of the present invention suppresses a viscosity decrease associated with high temperature heating also in food or beverage product.

[Table 8]

| Raw materials | Comparative Example 4-1 | Comparative Example 4-2 | Example 4-1 | Example 4-2 |
|---|---|---|---|---|
| Dark soy sauce | 5.5 | 5.5 | 5.5 | 5.5 |
| Sesame oil | 2.5 | 2.5 | 2.5 | 2.5 |
| Sweet cooking rice wine (hon-mirin) | 2 | 2 | 2 | 2 |
| Dietary salt | 1 | 1 | 1 | 1 |
| Sugar | 4 | 4 | 4 | 4 |
| Sodium L-glutamate | 0.7 | 0.7 | 0.7 | 0.7 |

(continued)

| Raw materials | Comparative Example 4-1 | Comparative Example 4-2 | Example 4-1 | Example 4-2 |
|---|---|---|---|---|
| SAN LIKE※ taste base※ A (modified) (* ) | 1.5 | 1.5 | 1.5 | 1.5 |
| Pepper SP-61524 (OA) (*) | 0.07 | 0.07 | 0.07 | 0.07 |
| Starch sample of Comparative Example 1-1-R | 3 | 0 | 0 | 0 |
| Starch sample of Comparative Example 1-1-NF | 0 | 3 | 0 | 0 |
| Starch sample of Example 1-1-A | 0 | 0 | 3 | 0 |
| Starch sample of Example 1-7-A | 0 | 0 | 0 | 3 |
| Water | Remaining part | Remaining part | Remaining part | Remaining part |
| Total amount | 100 | 100 | 100 | 100 |
| Viscosity (mPa·s) | 66.9 | 30.1 | 101 | 340.8 |
| (The unit of the formulation is mass%) | | | | |

[Production example: food containing modified starch]

(Sweet vinegar starchy sauce)

A sweet vinegar starchy sauce of the formulation of Table 9-1 was prepared by the following manufacturing method.

<Production method>

(1) The modified starch of Example 1-1-A was added to water, and the mixture was stirred at 85°C for 10 minutes.

(2) The remaining raw materials were added, dissolved with stirring for 5 minutes, and then filled into a heat-resistant pouch and sealed.

(3) The pouch was sterilized in a hot water bath at 85°C for 30 minutes.

[Table 9-1]

| Raw materials | parts by mass |
|---|---|
| Dark soy sauce | 10 |
| Sugar | 15 |
| Dietary salt | 1 |
| Brewed vinegar (acidity 10%) | 6 |
| Sesame oil | 0.5 |
| Modified starch of Example 1-1-A | 4 |
| SAN LIKE※ sauteed onion 9Y55E (*) | 0.1 |
| SAN LIKE※ taste base A (modified) (*) | 0.1 |

(continued)

| Raw materials | parts by mass |
|---|---|
| Pepper SP-61524(OA) (*) | 0.05 |
| Water | 63.25 |
| Total amount | 100 |

(White sauce)

A white sauce of formulations of Table 9-2 was prepared with the following production method.

<Production method>

(1) Roasted flour and the modified starch of Example 2-5 were added to water, and the mixture was heated and stirred at 80°C for 10 minutes.
(2) The remaining raw materials were added, and the mixture was stirred and dissolved.
(3) After correction of the evaporated water, the pouch was filled with the mixture and subjected to retort sterilization at 121°C for 10 minutes.

[Table 9-2]

| Raw materials | parts by mass |
|---|---|
| Milk | 10 |
| Fresh cream | 3 |
| Dietary salt | 1 |
| SAN SWEET※ SU-100 (*) | 0.01 |
| SAN LIKE※ Chicken extract 2822 E (*) | 0.1 |
| SAN LIKE※ taste base A (modified) (*) | 0.1 |
| Onion powder | 0.1 |
| White pepper powder | 0.05 |
| Roasted flour | 2.5 |
| Modified starch of Example 2-5 | 3 |
| Water | 80.14 |
| Total amount | 100 |

(White peach preparation)

A white peach preparation of the formulation of Table 9-3 was prepared by the following production method.

<Production method>

(1) Sugar and the modified starch of Example 1-1-A were added to a white peach puree and water, heated, and heated and stirred at 80°C for 10 minutes.
(2) The pH was adjusted to 3.8 using a 10% citric acid solution, and the total amount was corrected with evaporated water after adding flavor.
(3) The sample was filled into a pouch, and sterilized at 85°C for 30 minutes, and then cooled in a constant temperature water bath at 8°C for 2 hours.

[Table 9-3]

| Raw materials | parts by mass |
|---|---|
| White peach puree | 60 |
| Sugar | 20 |
| Modified starch of Example 1-1-A | 6 |
| Peach flavor NO. 21-4377 (*) | 0.05 |
| Water | 13.95 |
| Total amount | 100 |

(Batter and its cooked food)

Batter of the formulation of Table 9-4 was prepared to cook a tempura.

<Production method>

(1) The sweet potato was cut to a thickness of 1 cm.
(2) A batter liquid was prepared by adding raw materials to ice water.
(3) A sweet potato piece was coated with the batter liquid by being immersed in the batter liquid, and then subjected to cook in oil at 175°C for 3 minutes.

[Table 9-4]

| Raw materials | parts by mass |
|---|---|
| Weak flour | 33.2 |
| Baking powder | 0.5 |
| Modified starch of Example 1-2-A | 5 |
| Water | 61.3 |
| Total amount | 100 |

(Bread)

Bread of the formulation of Table 9-5 was prepared by the following production method.

<Production method>

(1) Hard flour, granulated sugar, skimmed milk powder, salt, and the modified starch of Example 2-5 were mixed in powder form.
(2) the above (1), butter, and water were put into a bread maker machine, and the dry yeast was put into a dedicated pocket and baked.

[Table 9-5]

| Raw materials | parts by mass |
|---|---|
| Hard flour | 225 |
| Unsalted butter | 30 |
| Granulated sugar | 17 |
| Skimmed milk powder | 6 |
| Dietary salt | 5 |
| Dry yeast | 2.8 |
| Modified starch of Example 2-5 | 25 |
| Water | 170 |
| Total amount | 480.8 |

(Udon noodle)

Udon noodle of the formulation shown in Table 9-6 were prepared by the following production method.

<Production method>

(1) Water was added to the all-purpose flour and the modified starch of Example 1-2-A, and the mixture was kneaded with a universal mixing stirrer for 10 minutes.
(2) Salt was added to (1), and the mixture was further mixed and kneaded for 5 minutes to obtain a lump of noodle dough.
(3) The noodle mass was roughly stretched by a noodle making machine to obtain a band-shaped dough containing a crumble-like noodle mass.
(4) Four of the dough of (3) were combined with the noodle making machine to obtain a sheet-like noodle strip, and then the noodle strip was rolled to a thickness of 3 mm and cut into a width of 3 mm to prepare raw udon noodles.
(5) The obtained raw udon noodles were boiled (at 98 to 100°C for 12 minutes), washed with water, and cooled to 10°C, and then immersed in acid (conditions: in a 0.5% aqueous solution of gluconodeltalactone (pH 2.9), for 30 seconds), packaged, sterilized (conditions: 90°C for 30 min with conveyor steamer), and cooled to 10°C, thereby obtaining chilled type boiled udon noodles (product pH = 4.8).

[Table 9-6]

| Raw materials | parts by mass |
|---|---|
| All-purpose flour | 90 |
| Modified starch of Example 1-2-A | 10 |
| Dietary salt | 3 |
| Water | 37 |
| Total amount | 140 |

(Cookie)

Udon noodle of the formulation shown in Table 9-7 were prepared by the following production method.

<Production method>

(1) The shortening and margarine were stirred using a universal mixing stirrer at 216 rpm until creamy.
(2) Granulated sugar was added, and the mixture was further stirred for 3 minutes.
(3) The carotene base was added to the whole egg mixed in advance in several portions and mixed.
(4) Pre-sieved weak flour, starch, skimmed milk powder, salt, and an expansion agent were added, and mixed with a rubber spatula.
(5) The dough was matured in a refrigerator for 30 minutes.
(6) The dough was stretched to a thickness of 5 mm, and punched out with a mold having a diameter of 32 mm.
(7) It was baked in an oven preheated to 180°C for 12 minutes.
(8) After the baked product was sufficiently cooled to room temperature, the product was stored in a sealed container containing a desiccant.

[Table 9-7]

| Raw materials | parts by mass |
|---|---|
| Shortening | 25 |
| Margarine | 25 |
| Granulated sugar | 35 |
| Whole egg | 15 |
| Carotene base NO. 80 (*) | 0.05 |
| Weak flour | 90 |
| Modified starch of Example 2-5 | 10 |
| Skimmed milk powder | 3 |
| Dietary salt | 0.5 |
| SAN OVER※ O-62 (*) | 1 |
| Total amount | 204.55 |

(Lact ice)

Lact ice of the formulations of Table 9-8 was prepared by the following production methods.

<Production method>

(1) The powder raw material was added while the starch syrup and water were stirred.
(2) After heating, coconut oil was added at 80°C, and the mixture was further stirred for 10 minutes.
(3) The total amount was corrected with water and homogenized by a high pressure homogenizer (200 kgf/cm$^2$).
(4) After cooling to 5°C, it was aged overnight and subjected to freezing after addition of flavor (overrun: 70%) .

[Table 9-8]

| Raw materials | parts by mass |
|---|---|
| Sugar | 11 |
| Starch syrup | 8 |
| Glucose | 3 |
| Coconut oil | 8 |
| Skimmed milk powder | 5.5 |
| Modified starch of Example 1-1-A | 1 |

(continued)

| Raw materials | parts by mass |
|---|---|
| HOMOGEN※ DM-S (*) | 0.2 |
| SAN BEST NN-305 (*) | 0.15 |
| Vanilla flavor No. 21-1838 (*) | 0.1 |
| Water | 63.05 |
| Total amount | 100 |

(Mayonnaise type dressing)

A mayonnaise type dressing of the formulations of Table 9-9 was prepared by the following production method.

<Production method>

(1) The modified starch of Example 1-2-A, sugar, sodium L-glutamate, and seasoning were added to water with stirring, and the mixture was heated at 90°C for 10 minutes.
(2) After cooling to room temperature, moisture was adjusted, and a sweetened egg yolk was added and stirred and mixed.
(3) A brewed vinegar, an apple vinegar, and a lemon juice were added and stirred and mixed.
(4) A salad oil was added little by little with stirring.
(5) The mixture was emulsified with a colloid mill with a clearance set to 200 μm.
(6) The mixture obtained was filled in a container.

[Table 9-9]

| Raw materials | parts by mass |
|---|---|
| Salad oil | 35 |
| Brewed vinegar (acidity 10%) | 2 |
| Apple vinegar (acidity 5%) | 3 |
| Concentrated lemon juice | 2 |
| 20% sweetened egg yolk | 12.5 |
| Dietary salt | 2.5 |
| Sodium L-glutamate | 0.5 |
| SAN LIKE※ Amino base NAG (*) | 0.3 |
| Modified starch of Example 1-2-A | 4 |
| Water | 38.2 |
| Total amount | 100 |

(Yoghurt)

Yoghurt of the formulations of Table 9-10 were prepared by the following production method.

<Production method>

(1) Sugar, the modified starch of Example 1-2-A was added to milk, heated to 70°C, and then the mixture was stirred for 10 minutes.
(2) The total amount was corrected with water, and the mixture was homogenized by a high-pressure homogenizer (150 kgf/cm$^2$).
(3) The mixture was heated and sterilized at 90°C for 10 minutes, and then cooled to about 40°C.
(4) As a starter, a commercially available yogurt was added in an amount of 3% of the total amount.
(5) The mixture was placed in an incubator at 40°C and fermented until the pH reached 4.6.
(6) The curd was stirred to crush it into a uniform state and aliquoted into a container.
(7) The mixture was allowed to stand at room temperature to cool down, and then cooled in a refrigerator at 5°C.

[Table 9-10]

| Raw materials | parts by mass |
|---|---|
| Milk | 35 |
| Sugar | 2 |
| Modified starch of Example 1-2-A | 3 |
| Water | 60 |
| Total amount | 100 |

(Coffee beverage)

A coffee beverage of the formulations of Table 9-11 was prepared by the following method.

<Production method>

(1) A powder mixture of sugar, the modified starch of Example 1-1-A, and an emulsifier was added to water and dissolved at 75°C for 10 minutes.
(2) After cooling to room temperature, milk and coffee extract were added.
(3) The pH was adjusted to 6.8 using sodium bicarbonate.
(4) The mixture was heated to 75°C, and homogenization treatment was performed (150 kgf/cm$^2$).
(5) After filling the mixture into the can, retort sterilization was performed at 123°C for 20 minutes.

[Table 9-11]

| Raw materials | parts by mass |
|---|---|
| Coffee extract (Bx. 3.0) | 50 |
| Milk | 10 |
| Sugar | 6 |
| Modified starch of Example 1-1-A | 0.2 |
| HOMOGEN※ No. 2115 (F) (*) | 0.17 |
| Water | 33.63 |
| Total amount | 100 |

(Rolled egg)

A coffee beverage of the formulations of Table 9-12 was prepared by the following method.

<Production method>

(1) Materials other than whole egg and modified starch were added to water, and the mixture was stirred for 10 minutes.
(2) The whole egg and the modified starch of Example 1-1-A were further added to (1) and mixed.
(3) (2) was poured in 3 portions into a frying pan heated to 170°C to prepare a fried egg.
(4) The sample was vacuum-packed, sterilized at 85°C for 30 minutes, then cooled with running water, refrigerated, and then stored.

[Table 9-12]

| Raw materials | parts by mass |
|---|---|
| Whole egg | 60 |
| Modified starch of Example 1-1-A | 3 |
| Soy sauce ※ | 1.3 |
| Sweet cooking rice wine (mirin) ※ | 1 |
| Dietary salt | 0.5 |
| SAN LIKE Japanese style stock L (*) | 0.5 |
| SAN LIKE Amino base NAG (*) | 0.1 |
| Trehalose | 2 |
| Water | 31.6 |
| Total amount | 100 |

[Measurement of water-insoluble dietary fiber and water-soluble dietary fiber of dietary fiber used in Test Example]

The amounts of the water-insoluble dietary fiber and the water-soluble dietary fiber were measured by the modified Prosky method for the citrus fiber, the apple-derived dietary fiber, the bamboo-derived dietary fiber, the pea-derived dietary fiber, and the sugar beet-derived dietary fiber used for the production of the modified starch of Test Examples 1 to 6. From the obtained results, the ratio between the water-insoluble dietary fiber and the water-soluble dietary fiber of each dietary fiber was determined as shown in Table 10.

[Table 10]

| Dietary fiber raw material used for production of modified starch | (Water-insoluble dietary fiber) | : | (Water-soluble dietary fiber) |
|---|---|---|---|
| Citrus fiber | 1 | : | 0.29 |
| Apple-derived dietary fiber | 1 | : | 0.22 |
| Bamboo-derived dietary fiber | 1 | : | 0.02 |
| Pea-derived dietary fiber | 1 | : | 0.03 |
| Sugar beet-derived dietary fiber | 1 | : | 2.13 |

## Claims

1. A method for producing a physically modified starch, comprising subjecting a mixture for heat treatment to dry heat treatment,

    wherein the mixture for heat treatment comprises (A) starch, (B) a plant-derived dietary fiber or a mushroom-derived dietary fiber, and 0 to 20 mass% of moisture, and
a pH at 25°C when 5 g of the mixture for heat treatment is suspended in 95 ml of water is 5 to 12.

**2.** The production method according to claim 1,
wherein the dry heat treatment is performed at a temperature of 100 to 200°C.

**3.** The production method according to claims 1 or 2, wherein the mixture for heat treatment further comprises (C) a basic compound.

**4.** The production method according to claim 3, further comprising

preparing an intermediate mixture comprising (A) the starch, (B) the plant-derived dietary fiber or the mushroom-derived dietary fiber, (C) the basic compound, and 20 to 99 mass% of moisture, and
drying the intermediate mixture.

**5.** The production method according to claim 3, further comprising allowing (B) the plant-derived dietary fiber or the mushroom-derived dietary fiber to coexist in the mixture for heat treatment without adding water.

**6.** The production method according to any one of claims 1 to 5, wherein (A) the starch comprises one kind or two or more kinds selected from the group consisting of corn starch, potato starch, tapioca starch, rice starch, wheat starch, sweet potato starch, mung bean starch, arrowroot starch, and sago starch, and waxy starch thereof.

**7.** The production method according to any one of claims 1 to 6, wherein (B) the plant-derived dietary fiber is one kind or two or more kinds selected from the group consisting of a citrus-derived dietary fiber, an apple-derived dietary fiber, an orange-derived dietary fiber, a pea-derived dietary fiber, a soybean-derived dietary fiber, a carrot-derived dietary fiber, a tomato-derived dietary fiber, a bamboo-derived dietary fiber, a sugar beet-derived dietary fiber, a seed coat-derived dietary fiber, a corn-derived dietary fiber, a wheat-derived dietary fiber, a barley-derived dietary fiber, a rye-derived dietary fiber, and a konjac-derived dietary fiber.

**8.** The production method according to any one of claims 1 to 7, wherein (C) the basic compound is one kind or two or more kinds selected from the group consisting of hydroxides, carbonates, hydrogencarbonates, and organic acid salts of alkali metals, and hydroxides, carbonates, hydrogencarbonates, and organic acid salts of alkaline earth metals.

**9.** The production method according to any one of claims 1 to 8, wherein a content of the component (B) with respect to 100 parts by mass of the component (A) in the mixture for heat treatment is 0.5 parts by mass or more and less than 30 parts by mass.

**10.** A method for suppressing viscosity decrease at a time of gelatinization of starch or by subsequent high temperature heating, or imparting viscosity stability after gelatinization, comprising
allowing an effective amount of a plant-derived dietary fiber or a mushroom-derived dietary fiber to coexist with starch and subjecting a mixture to dry heat treatment.

**11.** An agent for physically modifying starch by dry heat treatment, comprising an effective amount of a plant-derived dietary fiber or a mushroom-derived dietary fiber.

**12.** A physically modified starch comprising a plant-derived dietary fiber or a mushroom-derived dietary fiber, wherein when a viscosity of a water suspension of 4.8 mass% of the starch is measured under first conditions using a rapid viscoanalyzer, a difference between a maximum value of viscosity Va during a period from a gelatinization period to a high temperature holding period and a minimum value of viscosity Vb during a cooling period, that is, Va-Vb, is 100 mPa·s or less,
the first conditions are that

(1) a sample temperature is held at 50°C for a hydration period of 0 to 60 seconds,

raised at 0.203°C/sec for the gelatinization period of 60 to 282 seconds,
held at 95°C for the high temperature holding period of 282 to 432 seconds,
lowered at 0.197°C/sec for the cooling period of 432 to 660 seconds, and

held at 50°C for a cool holding period of 660 to 780 seconds, and

(2) a rotating speed of a paddle is 960 rpm from 0 to 10 seconds, and 160 rpm after 10 seconds.

**13.** The physically modified starch according to claim 12, wherein an initial viscosity increase rate (Vr) is 0.02/s or less when the viscosity of the water suspension of 4.8 mass% of the starch is measured under the first conditions using the rapid viscoanalyzer.

**14.** A physically modified starch comprising a plant-derived dietary fiber or a mushroom-derived dietary fiber, wherein when a viscosity of a water suspension of 4.8 mass% of the starch is measured under second conditions using a rapid viscoanalyzer, a difference between a maximum value of viscosity Va during a period from a gelatinization period to a high temperature holding period and a minimum value of viscosity Vb during a cooling period, that is, Va-Vb, is 150 mPa·s or less,
the second conditions are that

(1) a sample temperature is held at 50°C for a hydration period of 0 to 60 seconds,

raised at 0.194°C/sec for the gelatinization period of 60 to 420 seconds,
held at 120°C for the high temperature holding period of 420 to 1020 seconds, and
lowered at 0.194°C/sec for the cooling period of 1020 to 1380 seconds, and

(2) a rotating speed of a paddle is 960 rpm from 0 to 10 seconds, and 160 rpm after 10 seconds.

**15.** The physically modified starch according to claim 14, wherein an initial viscosity increase rate (Vr) is 0.02/s or less when the viscosity of the water suspension of 4.8 mass% of the starch is measured under the second conditions using the rapid viscoanalyzer.

**16.** A physically modified starch comprising a plant-derived dietary fiber or a mushroom-derived dietary fiber, wherein a relative value A of viscosities before and after retort sterilization treatment defined below is 80% or more and a relative value B of viscosities during storage after the retort sterilization treatment is 85% to 125% in a viscosity of a gelatinized starch paste obtained from a water suspension containing 5 mass% of the starch:

relative value A of viscosities before and after retort sterilization treatment (%) = $\eta_2/\eta_1 \times 100$,
relative value B of viscosities during storage after retort sterilization treatment (%) = $\eta_3/\eta_2 \times 100$; wherein
the water suspension containing 5 mass% of the physically modified starch is heated in a hot water bath while being stirred, kept at 90°C for 10 minutes after reaching 90°C, and then cooled in a constant temperature water bath at 20°C for 1 hour, and then a viscosity measured at 60 rpm using a B-type rotational viscometer is defined as $\eta_1$,
the water suspension containing 5 mass% of the physically modified starch is heated in a hot water bath while being stirred, kept at 90°C for 10 minutes after reaching 90°C, further subjected to a retort sterilization treatment under conditions of 121°C for 20 minutes, allowed to cool at 20°C for 1 day, and then a viscosity measured at 60 rpm using a B-type rotational viscometer is defined as $\eta_2$,
the water suspension containing 5 mass% of the physically modified starch is heated in a hot water bath while being stirred, kept at 90°C for 10 minutes after reaching 90°C, further subjected to a retort sterilization treatment under conditions of 121°C for 20 minutes, allowed to cool at 20°C for 1 day, then stored in a constant temperature chamber at 5°C for 1 week (7 days), and temperature-controlled by being immersed in a constant temperature water bath at 20°C for 1 hour, and then a viscosity measured at 60 rpm using a B-type rotational viscometer is defined as $\eta_3$.

**17.** A formulation comprising the physically modified starch according to any one of claims 12 to 16.

**18.** A food or beverage product comprising the physically modified starch according to any one of claims 12 to 16.

FIG. 1A

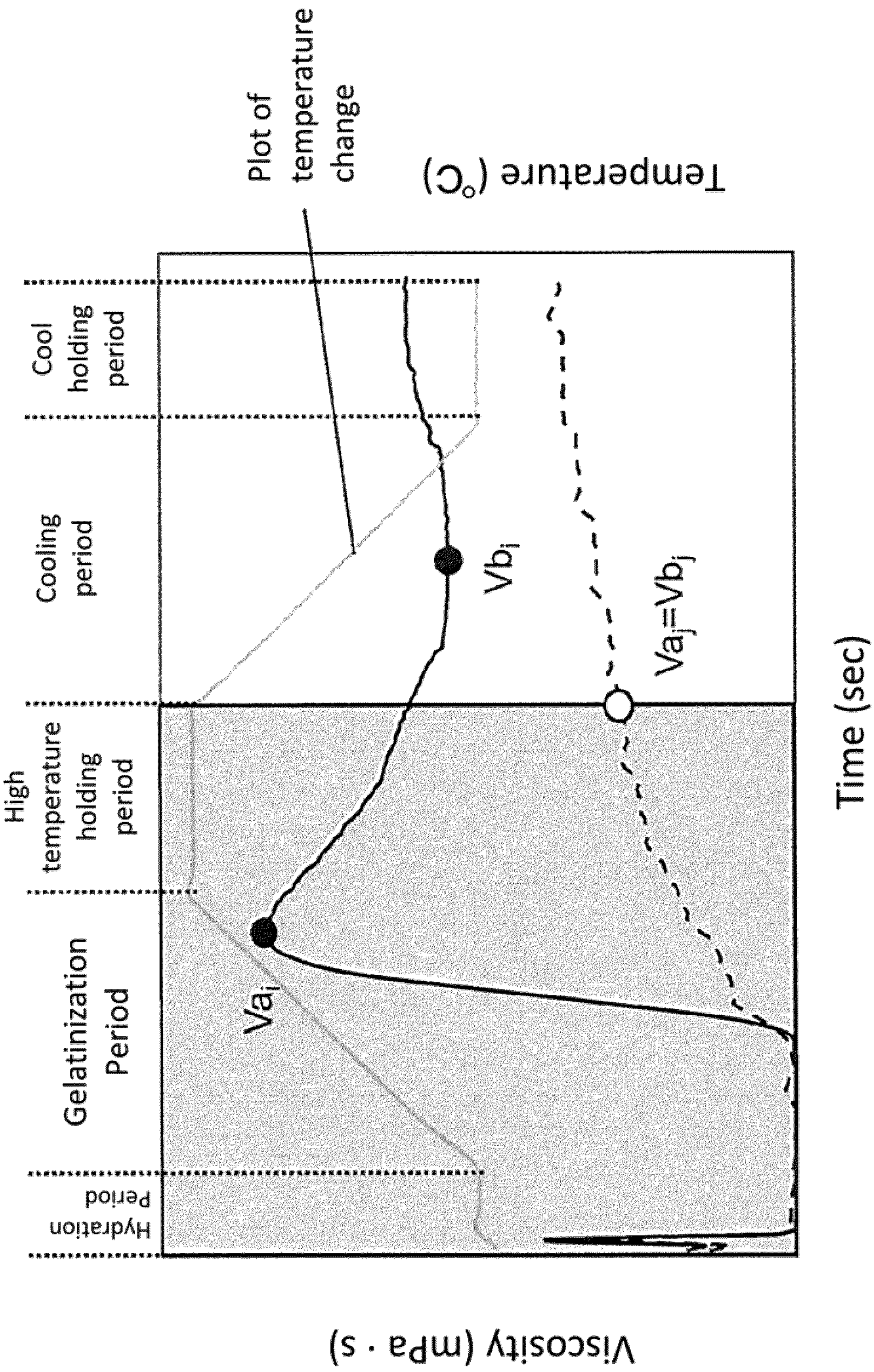
Hydration Period
Gelatinization Period
High temperature holding period
Cooling period
Cool holding period
Plot of temperature change
Temperature (°C)
Viscosity (mPa · s)
Time (sec)
$Va_i$
$Vb_i$
$Va_j$=$Vb_j$

FIG. 1B

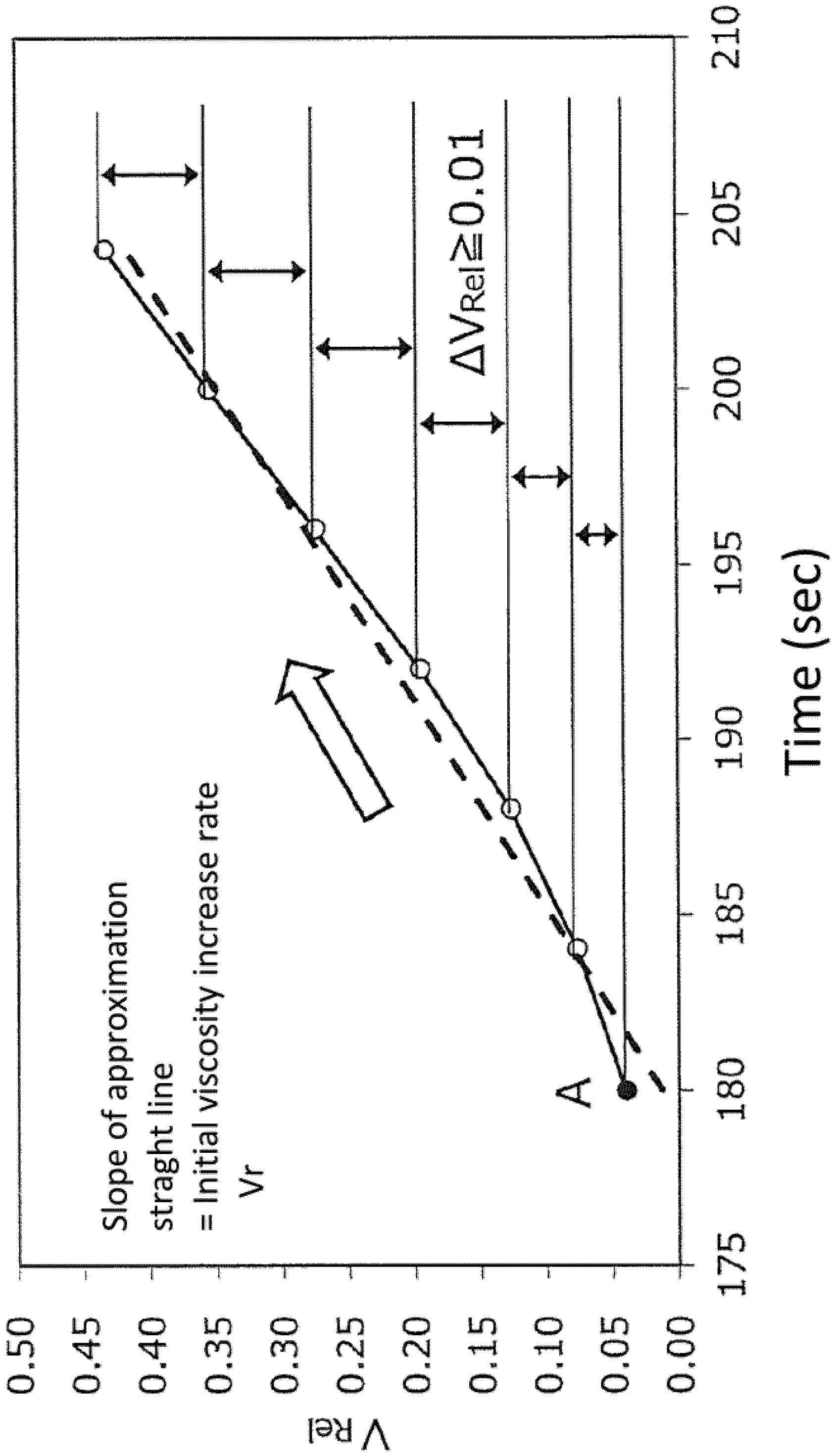
Slope of approximation straght line
= Initial viscosity increase rate
Vr
A
ΔV$_{Rel}$≧0.01
V Rel
Time (sec)
0.00
0.05
0.10
0.15
0.20
0.25
0.30
0.35
0.40
0.45
0.50
175
180
185
190
195
200
205
210

FIG. 2

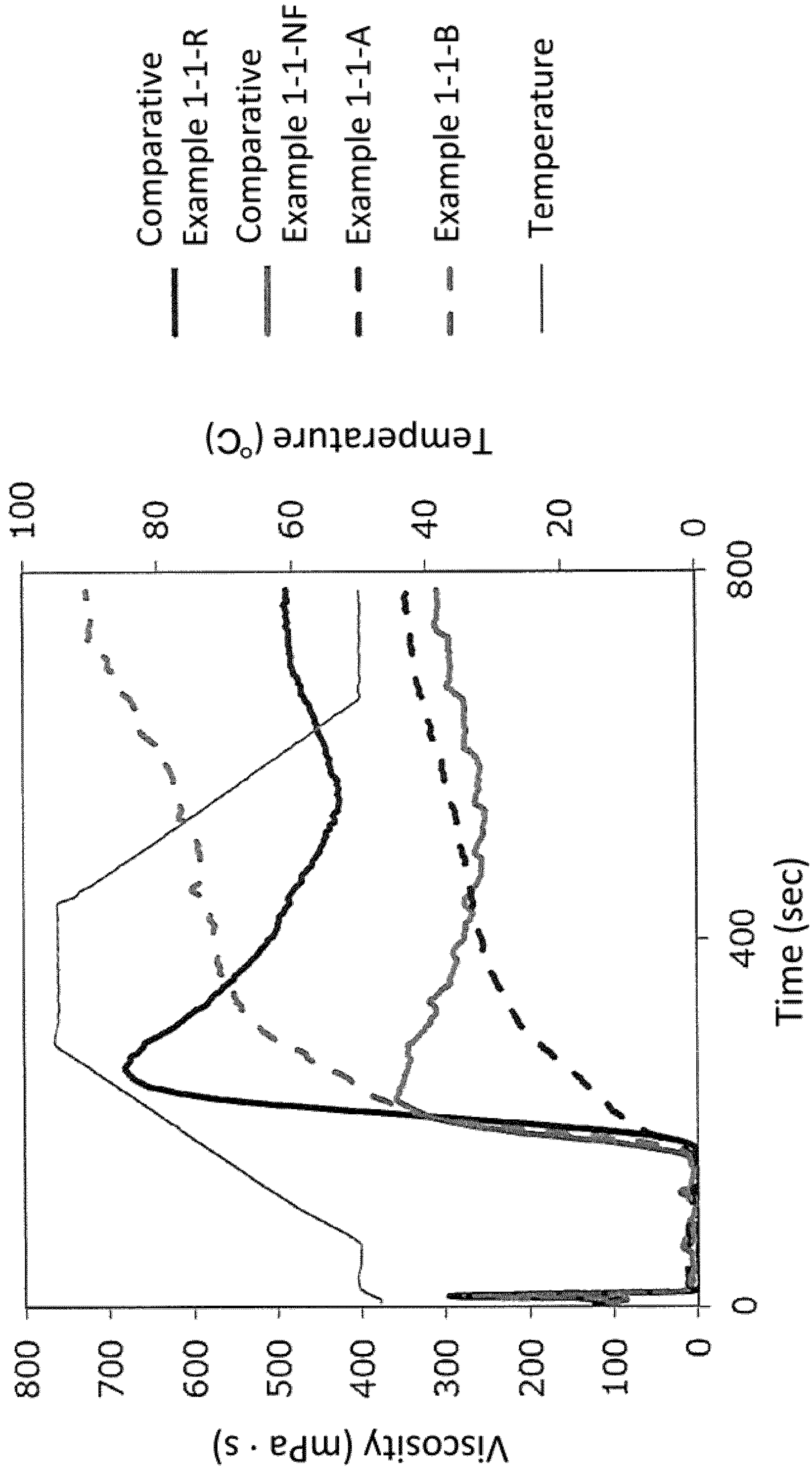
Viscosity (mPa · s)
Temperature (°C)
Time (sec)
Comparative Example 1-1-R
Comparative Example 1-1-NF
Example 1-1-A
Example 1-1-B
Temperature
0
100
200
300
400
500
600
700
800
0
20
40
60
80
100
0
400
800

FIG. 3

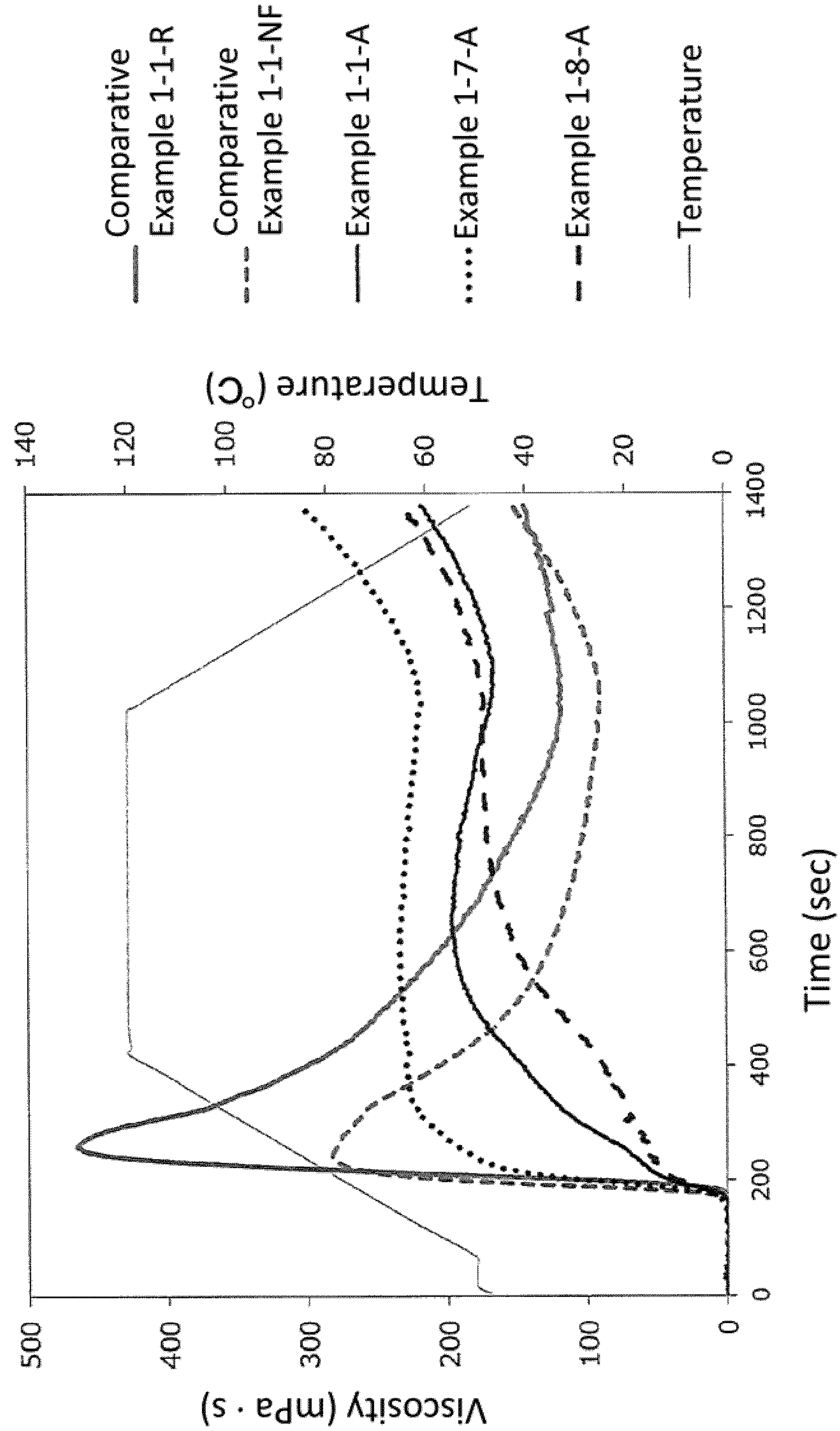
Comparative Example 1-1-R
Comparative Example 1-1-NF
Example 1-1-A
Example 1-7-A
Example 1-8-A
Temperature
Viscosity (mPa · s)
Temperature (°C)
Time (sec)
0
100
200
300
400
500
0
20
40
60
80
100
120
140
0
200
400
600
800
1000
1200
1400

FIG. 4

| | After treatment of RVA profile 1 | After treatment of RVA profile 2 |
|---|---|---|
| Comparative Example 1-1-R | 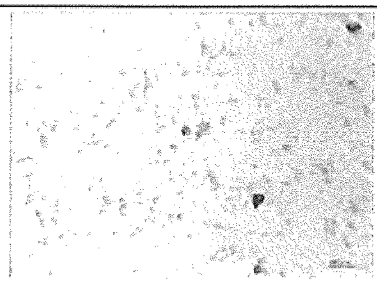 | 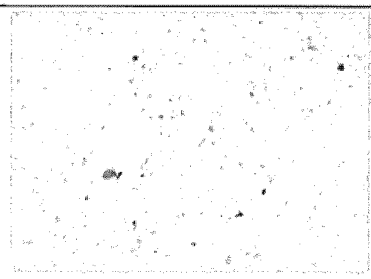 |
| Comparative Example 1-1-NF | 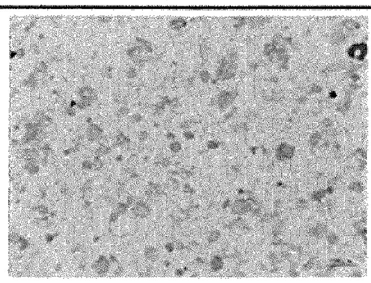 | 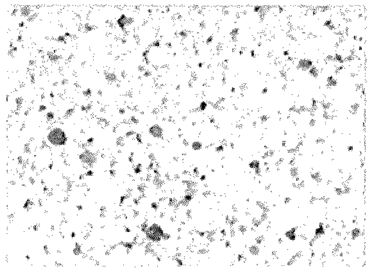 |
| Example 1-1-A | 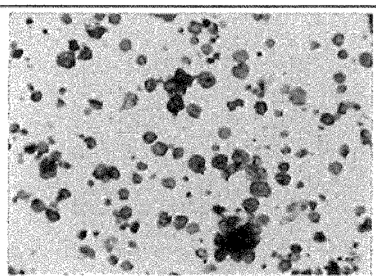 | 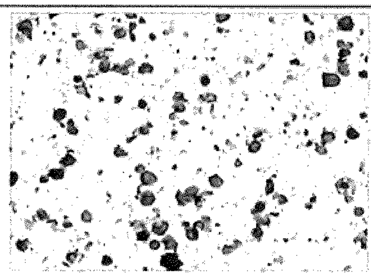 |
| Example 1-7-A | 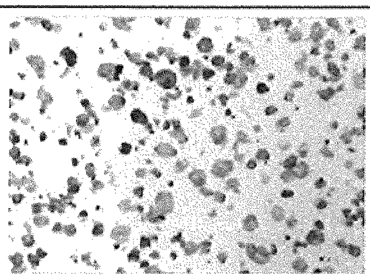 | 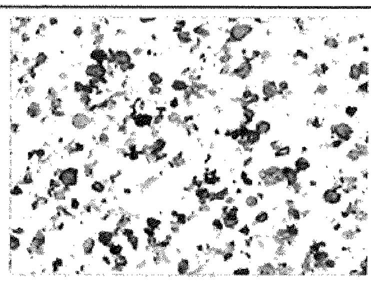 |
| Example 1-8-A | 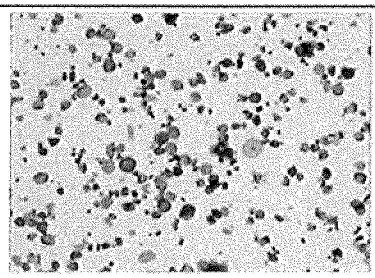 | 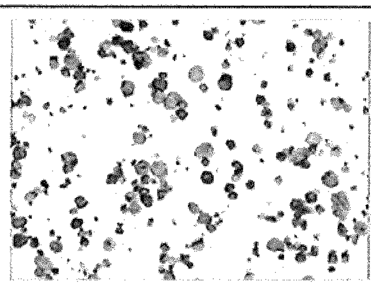 |

**INTERNATIONAL SEARCH REPORT**

International application No.
PCT/JP2020/037627

A. CLASSIFICATION OF SUBJECT MATTER
Int. Cl. C08B30/14(2006.01)i, A23L29/212(2016.01)i
FI: A23L29/212, C08B30/14

According to International Patent Classification (IPC) or to both national classification and IPC

B. FIELDS SEARCHED

Minimum documentation searched (classification system followed by classification symbols)
Int. Cl. G08B30/14, A23L29/212

Documentation searched other than minimum documentation to the extent that such documents are included in the fields searched
Published examined utility model applications of Japan 1922-1996
Published unexamined utility model applications of Japan 1971-2020
Registered utility model specifications of Japan 1996-2020
Published registered utility model applications of Japan 1994-2020

Electronic data base consulted during the international search (name of data base and, where practicable, search terms used)
JSTPlus/JMEDPlus/JST7580 (JDreamIII)

C. DOCUMENTS CONSIDERED TO BE RELEVANT

| Category* | Citation of document, with indication, where appropriate, of the relevant passages | Relevant to claim No. |
|---|---|---|
| X<br>A | SUDHA M. L., et al., Studies on pasting and structural characteristics of thermally treated wheat germ, Eur Food Res Technol, 2007, vol. 225, pp. 351-357, in particular, Materials and methods, table 1, in particular, Materials and methods, table 1 | 1-2, 6-7, 9-10, 12-18<br>3-5, 8, 11 |
| X<br>A | JP 9-12601 A (NATIONAL STARCH AND CHEMICAL INVESTMENT HOLDING CORPORATION) 14 January 1997, claims 1, 15, paragraph [0044], claims 1, 15, paragraph [0044] | 1-2, 6-7, 9-10, 12-18<br>3-5, 5, 11 |
| X<br>A | KR 10-2018-0055026 A (REPUBLIC OF KOREA(MANAGEMENT RURAL DEVELOPMENT ADMINISTRATION)) 25 May 2018, claims 1-2, examples 2-3, claims 1-2, examples 2-3 | 1-2, 6-7, 9-18<br>3-5, 8 |

☐ Further documents are listed in the continuation of Box C.  ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 12.11.2020 | 24.11.2020 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application No.
PCT/JP2020/037627

| Patent Documents referred to in the Report | Publication Date | Patent Family | Publication Date |
|---|---|---|---|
| JP 9-12601 A | 14.01.1997 | US 5593503 A<br>claim 1, example 1<br>US 5902410 A<br>EP 747397 A2 | |
| KR 10-2018-0055026 A | 25.05.2018 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2005054028 A
- WO 2018216748 A
- WO 2016114201 A
- JP 2019195308 A
- JP 2019041735 A